# EUROPEAN PATENT SPECIFICATION

(11) **EP 4 161 626 B1**
(45) Date of publication and mention of the grant of the patent: **21.08.2024**
(21) Application number: 21732572.9
(22) Date of filing: 09.06.2021
(51) Int. Cl.: A61M 27/00, A61F 2/04, A61B 17/32, A61B 18/14, A61F 2/88, A61F 2/92, A61B 17/3207, A61B 17/22, A61B 90/00

(54) **DEVICES FOR URETHRAL TREATMENT**
VORRICHTUNGEN ZUR HARNRÖHRENBEHANDLUNG
DISPOSITIFS DE TRAITEMENT URÉTRAL

(30) Priority: 09.06.2020 US 202063036548 P
(43) Date of publication of application: 12.04.2023
(73) Proprietor: Proarc Medical Ltd., 2017400 Doar-Na Misgav (IL)
(72) Inventor: HEFTMAN, Gilad, 3750433 Pardes Hana-Karkur (IL); HASON, Eran, 3705403 Pardes Hana-Karkur (IL); NITSAN, David, 6209236 Tel Aviv (IL)
(74) Representative: Boult Wade Tennant LLP
(86) International application number: PCT/IB2021/055063
(87) International publication number: WO 2021/250588

(56) References cited:
- WO-A2-2008/142677
- US-A1- 2016 000 455
- US-A1- 2018 344 995
- US-A1- 2019 381 291

## Description

### FIELD OF EMBODIMENTS OF THE INVENTION

The present invention, in some embodiments thereof, relates to devices for treatment of intrabody lumens, and, more particularly, but not exclusively, to devices for dilating and/or assisting in dilation and/or maintaining dilation of the urethra to relieve obstruction resulting, for example from benign prostatic hyperplasia (BPH).

### BACKGROUND

It is common for the prostate gland to become enlarged as a man ages. As a male matures, the prostate goes through two main periods of growth, first early in puberty, and then again at later age, when the growth begins again, and continues on through life. One of the effects of this continued growth can be pressure on the urethra, the passage through which urine passes from the bladder and the penis.

The urethra is surrounded by the prostate for part of its length. Within the confines of the prostate, the urine flows through a passage having a generally triangular cross-section. As the prostate enlarges, the layer of tissue surrounding the prostate restricts the prostate from expanding outward, causing the prostate to constrict the urethral passage. The condition of an enlarged, non-cancerous prostate is called benign prostatic hyperplasia (BPH).

Though the prostate continues to grow during most of a man's life, benign prostatic hyperplasia rarely causes symptoms before age 40, but more than half of men in their sixties and as many as 90 percent in their seventies and eighties have some symptoms. BPH can make it difficult to completely empty the bladder and is associated with other urinary system problems.

US 2018/344995 A1 discloses an apparatus comprising a device for treatment of a urethra that is constricted due to benign prostatic hyperplasia.

### SUMMARY

The invention is defined by claim 1. Further embodiments of the invention are defined by the dependent claims. Methods as such are not claimed.

In some embodiments of the present disclosure, apparatus and methods for treatment of a urethra that is constricted due to benign prostatic hyperplasia (BPH), are provided. Typically, the apparatus includes a device having a delivery tool, which has a proximal portion and a distal portion. The distal portion of the delivery tool is configured to be advanced to a location in an area of the urethra that is to be treated due to enlargement of the prostate and consequent constriction of the area to be treated. In some embodiments the distal portion includes an operational head having an expandable element, a tissue cutter, and an implant. The expandable element e.g., a balloon, expands within the urethra such as to enlarge the urethra and dilate the constricted area. The tissue cutter is typically disposed outside of an outer surface of the expandable element and forms a cut in an inner surface of the urethra, subsequently to the expandable element having expanded in the urethra.

The implant is then released into the cut within the inner surface of the urethra and implanted in tissue of the prostate surrounding the cut in the urethra, to maintain the urethra in a dilated state. In accordance with some embodiments of the present invention, the implant comprises a shape-memory material, being shape set such that in an unconstrained configuration of the implant, the implant is substantially straight. The implant is shaped to define two end sections and a middle section disposed between the two end sections, and the shape memory-material is shape set such that in an unconstrained configuration of the implant, at least the middle section is substantially straight. (In some instances, such an implant is described herein as a "quasi-straight implant".)

In accordance with some embodiments of the present disclosure, upon implantation of the implant in the cut formed in the urethra, the implant is constrained into a curved shape by the tissue in which the implant is implanted. Due to the tendency of the shape-memory material to return to its pre-set generally straight configuration, the implant applies radially outward pressure to the tissue. By being shape set in the generally straight configuration, the implant typically dilates the urethra more effectively than if the implant were to be shape set such as to define a less generally straight shape.

In accordance with some embodiments of the present disclosure, the device further includes one or more implant carrier arms extending from a shaft of the delivery tool and being disposed outside the outer surface of the expandable element. The implant carrier arms typically release the implant into the cut, subsequently to the tissue cutter forming the cut in the inner surface of the urethra. In some embodiments, one or more implant holders are coupled to the implant carrier arm and the implant is disposed between the implant carrier arm and the implant holder such that the implant is held in place during delivery of the implant. Subsequently to the tissue cutter forming the cut in the urethra, the implant holders are retracted proximally with respect to the implant carrier arms such that the implant is released by the implant carrier arms into a cut within the urethra, to maintain the urethra in a dilated state.

There is therefore provided in accordance with some embodiments of the present invention, apparatus including:
a device for treatment of a urethra that is constricted due to benign prostatic hyperplasia (BPH), the device including:
   a delivery tool having a proximal portion and a distal portion, the distal portion configured to be advanced to a location in an area of the urethra that is to be treated, and the distal portion including:
   an expandable element that is configured to expand within the urethra such as to enlarge the urethra in the area to be treated, the expandable element defining an outer surface;
   a tissue cutter disposed outside the outer surface of the expandable element, the tissue cutter configured to form a cut in an inner surface of the urethra, subsequent to the expandable element having expanded such as to dilate the urethra; and
   an implant that includes a shape-memory material, and being configured to maintain the urethra in a dilated state, by being implanted within the cut in the inner surface of the urethra,
   the implant including two end sections and a middle section disposed between the two end sections, and the shape memory-material being shape set such that in an unconstrained configuration of the implant, the middle section is substantially straight.

In some embodiments, the implant is configured to be implanted within the cut in the urethra such that the middle section of the implant is constrained into a curved shape.

In some embodiments, a length of the middle section of the implant is at least 50% of a length of the implant.

In some embodiments, a length of the middle section of the implant is at least 60% of a length of the implant.

In some embodiments, a length of the middle section of the implant is at least 70% of a length of the implant.

In some embodiments, the implant is configured to be maintained in a spiral shape, while the implant is disposed within the delivery tool

In some embodiments, the implant has a length of 40 - 80 mm.

In some embodiments, the implant has a thickness of 0.01 - 1 mm.

In some embodiments, the implant has a width of 0.5 - 4 mm.

In some embodiments, the implant is configured to apply pressure within the cut in the urethra of from about 25gr to about 500gr.

In some embodiments, the tissue cutter is configured to form the cut having a depth of 2 - 10 mm.

In some embodiments, the tissue cutter has a length of 3 - 10 mm.

In some embodiments, the tissue cutter is configured to form the cut at an angle of 90 degrees with respect to a longitudinal axis of the delivery tool.

In some embodiments, the tissue cutter is configured to form the cut at an angle other than 90 degrees with respect to a longitudinal axis of the delivery tool.

In some embodiments, the tissue cutter is configured to form the cut at an angle of from about 45 degrees to about 89 degrees with respect to a longitudinal axis of the delivery tool.

In some embodiments, the expandable element is configured to be inflated to an internal volume of 3 -15mm³.

In some embodiments, the expandable element is configured to be inflated to define a diameter of 5 - 35 mm.

In some embodiments, the expandable element has a length of 3 - 100 mm.

In some embodiments, the expandable element is configured to be inflated to an internal pressure of 1- 20 atm.

In some embodiments, the expandable element includes a balloon.

In some embodiments, the apparatus further includes one or more implant carrier arms extending from a shaft of the delivery tool and being disposed outside the outer surface of the expandable element.

In some embodiments, subsequently to the tissue cutter forming the cut in the inner surface of the urethra, the one or more implant carrier arms are configured to release the implant into the cut.

In some embodiments, the apparatus further includes one or more implant holders coupled to the implant carrier arms, and:
the implant is configured to be disposed between the implant carrier arm and the implant holder such that the implant is held in place by the implant carrier arm and the implant holder; and
the implant holders being configured to be retracted proximally with respect to the implant carrier arms such that the implant is released by the implant carrier arms into a cut within the urethra, to thereby maintain the urethra in a dilated state.

In some embodiments, the delivery tool further includes an optical element, and the one or more implant carrier arms and the expandable element are configured so as to allow the optical element to visualize the urethra.

There is yet further provided in accordance with some embodiments of the present disclosure, an example method for treating a urethra that is constricted due to benign prostatic hyperplasia (BPH), including:
identifying a constricted area of the urethra requiring treatment;
inserting into the urethra a delivery tool that includes an expandable element that defines an outer surface, and a tissue cutter disposed outside the outer surface of the expandable element;
using the delivery tool, delivering, to the identified constricted area of the urethra, an implant that includes a shape-memory material and has two end sections and a middle section disposed between the two end sections, the shape memory-material being shape set such that in an unconstrained configuration of the implant, the middle section is substantially straight;
expanding the urethra by expanding the expandable element in the identified constricted area of the urethra;
subsequently, forming a cut in an inner surface of the urethra using the tissue cutter; and
releasing the implant into the cut to maintain the urethra in a dilated state.

In some embodiments, delivering the implant to the identified constricted area of the urethra includes delivering the implant while the implant is compressed into a spiral configuration.

In some embodiments, releasing the implant into the cut to maintain the urethra in a dilated state, includes releasing the implant into the cut while the middle section of the implant is constrained into a curved shape.

In some embodiments, the delivery tool further includes one or more implant carrier arms extending from a shaft of the delivery tool and being disposed outside the outer surface of the expandable element and delivering the implant to the identified constricted area of the urethra includes delivering the implant while the implant is in a removably coupled state with respect to the one or more implant carrier arms.

In some embodiments, releasing the implant into the cut includes releasing the implant from the one or more implant carrier arms.

In some embodiments, the delivery tool further includes one or more implant holders coupled to the implant carrier arms and delivering the implant to the identified constricted area of the urethra includes delivering the implant while the implant is held in place with respect to the one or more implant carrier arms by being disposed between the one or more implant carrier arms and the one or more implant holders.

In some embodiments, releasing the implant into the cut includes retracting one of the one or more implant holders proximally with respect to one of the one or more implant carrier arms.

In some embodiments, releasing the implant includes releasing a distal portion of the implant prior to expanding the expandable element and releasing a proximal portion of the implant subsequently to expanding the expandable element.

There is still further provided in accordance with some embodiments of the present disclosure, apparatus including:
a device for treatment of a urethra that is constricted due to benign prostatic hyperplasia (BPH), the device including:
a delivery tool having a proximal portion and a distal portion, the distal portion configured to be advanced to a location in an area of the urethra that is to be treated and the distal end including:
   one or more implant carrier arms extending from a shaft of the delivery tool; and
   one or more implant holders coupled to the one or more implant carrier arms; and
an implant configured to be disposed between the one or more implant carrier arms and the one or more implant holders such that the implant is held in place by the one or more implant carrier arms and the one or more implant holders,
   the one or more implant holders being configured to be retracted proximally with respect to the one or more implant carrier arms such that the implant is released from the one or more implant carrier arms into a cut within the urethra, to thereby maintain the urethra in a dilated state.

In some embodiments, the implant is held in a spiral configuration between the one or more implant carrier arms and the one or more implant holders.

In some embodiments, the one or more implant holders are configured to be retracted proximally to release a distal end of the implant prior to releasing a proximal end of the implant.

In some embodiments, the apparatus further includes a tissue cutter configured to form a cut in an inner surface of the urethra, and the implant is configured to be released into the cut.

In some embodiments, the apparatus further includes an optical element, and the one or more implant carrier arms and the expandable element are configured so as to allow the optical element to visualize the urethra.

In some embodiments, the apparatus further includes an expandable element that is configured to expand within the urethra such as to expand the urethra in the area to be treated.

In some embodiments, one of the one or more implant holders is configured to be retracted proximally to release a distal end of the implant prior to expanding of the expandable element, and a proximal end of the implant is configured to be released subsequently to expanding of the expandable element.

There is still further provided in accordance with some embodiments of the present disclosure, an example method for treating a urethra that is constricted due to benign prostatic hyperplasia (BPH), including:
identifying a constricted area of the urethra requiring treatment;
inserting into the urethra a longitudinal delivery tool that includes:
   an expandable element that defines an outer surface,
   a tissue cutter disposed outside the outer surface of the expandable element,
   one or more implant carrier arms extending from a shaft of the delivery tool, and
   one or more implant holders coupled to the implant carrier arms, and
   an implant disposed between the one or more implant carrier arms and the one or more implant holders such that the implant is held in place by the one or more implant carrier arms and the one or more implant holders;
delivering the implant for deployment at the identified constricted area of the urethra;
releasing a distal portion of the implant by retracting one of the one or more implant holders proximally with respect to the implant carrier arm;
expanding the urethra using the expandable element at the identified constricted area of the urethra;
forming a cut in the inner surface of the urethra; and
releasing a proximal portion of the implant by retracting one of the one or more implant holders proximally with respect to one of one or more implant carrier arms, thereby releasing the implant into the cut.

In some embodiments, delivering the implant for deployment at the identified constricted area of the urethra includes delivering the implant while the implant is held in a spiral configuration between the one or more implant carrier arms and the one or more implant holders.

In some embodiments, releasing the distal portion of the implant includes releasing the distal portion of the implant prior to expanding the expandable element and releasing the proximal portion of the implant includes releasing the proximal portion subsequently to expanding the expandable element.

There is still further provided in accordance with some embodiments of the present disclosure, an operational head for implantation system for dilating a urethra at least partially obstructed by an enlarged prostate including:
a. a quasi-straight implant;
b. at least two arms connected to a shaft and including implant holders to which the quasi-straight implant is removably attached;
c. an expandable body surrounded by the at least two arms and the quasi-straight implant is wrapped around the expandable element;
d. an optical element extending from the handle inside the shaft;
the at least two arms and the expandable element are configured so as to allow the optical element to visualize the urethra.

In some embodiments, the implant is configured to be at least partially embedded through the wall of an enlarged portion of the prostate surrounding a constricted portion of the urethra.

In some embodiments, the quasi-straight implant includes pad-like endings at each end.

In some embodiments, the quasi-straight implant includes one or more protrusions located at each end configured to stabilize the quasi-straight implant once implanted inside the cut.

In some embodiments, the quasi-straight implant includes a length of from about 40mm to about 80mm.

In some embodiments, the quasi-straight implant includes a thickness of from about 0.01mm to about 1mm.

In some embodiments, the quasi-straight implant includes a width of from about 0.5mm to about 4mm.

In some embodiments, the quasi-straight implant includes a middle straight section and two slightly curved end sections.

In some embodiments, the quasi-straight implant is configured to apply forces on the wall of from about 25gr to about 500gr.

In some embodiments, the at least two arms include a sleeve that partially covers the arms.

In some embodiments, the operational head include three arms.

In some embodiments, the at least two arms are configured to perform inward-outward movements, which accompany the inflation deflation if the expandable body.

In some embodiments, a relationship of width/thickness of the at least two arms provide the at least two arms with the necessary strength to resist bending due to rotational movements of the shaft.

In some embodiments, the implant holders are covered by the sleeve.

In some embodiments, the implant holders are configured to move proximally and distally in relation to the at least two arms.

In some embodiments, the operational head further includes a tissue cutter extending from a handle to the operational head and configured to perform a cut in the wall, the cut being the place where the implant is to be at least partially embedded.

In some embodiments, the cutter includes an L-shape operating configuration.

In some embodiments, the cutter is configured to cut by rotating the shaft.

In some embodiments, the cutter is configured to perform a cut having a depth of from about 2mm to about 10mm.

In some embodiments, the tissue cutter includes a length of from about 3mm to about 10mm.

In some embodiments, the cutter is connected to an electrical or electromechanical energy.

In some embodiments, the cutter includes a distal end including a T-like shape.

In some embodiments, the cutter includes a distal end including a rounded shape.

In some embodiments, the cutter is configured to deployed and/or inserted from the shaft by a user.

In some embodiments, the cutter performs a cut at an angle of 90 degrees in relation to the longitudinal axis of the device.

In some embodiments, the cutter performs a cut at an angle other than 90 degrees in relation to the longitudinal axis of the device.

In some embodiments, the cutter performs a cut at an angle of from about 45 degrees to about 89 degrees in relation to the longitudinal axis of the device.

In some embodiments, the expandable body includes an internal volume of from about 3mm³ to about 15mm³.

In some embodiments, the expandable body includes a diameter of from about 5mm to about 35mm.

In some embodiments, the expandable body includes a length of from about 3mm to about 100mm.

In some embodiments, the expandable body can be inflated to an internal pressure of from about 1atm to about 20atm.

In some embodiments, the expandable body includes a balloon.

In some embodiments, the expandable body is a balloon.

In some embodiments, the expandable body is made of elastic materials.

In some embodiments, the expandable body is in communication with a tube extending from the handle, the tube configured to transport the material that causes the expandable body to be inflated/deflated.

In some embodiments, the expandable body is enclosed in a jacket configured to protect the expandable body.

In some embodiments, the jacket limits the inflation of the expandable body.

In some embodiments, a distal end of the optical element is located before a proximal end of the operational head.

In some embodiments, the distal end of the optical element is located at about ±2mm of a distal end of the shaft.

In some embodiments, the optical element includes a field of view of from about 0 degrees to about ±120 degrees.

In some embodiments, the handle includes one or more controllers configured to actuate the at least two motors.

In some embodiments, the handle includes one or more controllers configured to synchronize the actuation of the at least two motors.

In some embodiments, the at least two motors are configured to operate at a velocity of from about 1RPM to about 1000RPM.

In some embodiments, the at least two motors are configured to provide a force of from about 1Kg to about 5Kg.

In some embodiments, the at least two motors are configured to provide linear movements and/or rotational movements to one or more of the elongated body and the shaft.

In some embodiments, the rotational movement includes a velocity of from about 1 rotation per second to about 1 rotation per 3 seconds.

In some embodiments, the rotational movement includes a rotational force (torque) of from about 50Nmm to about 300Nmm.

In some embodiments, the linear movement includes a velocity of from about 1mm/s to about 10mm/s.

In some embodiments, the elongated body includes an internal diameter of from about 3mm to about 20mm.

In some embodiments, the shaft includes an internal diameter of from about 3mm to about 20mm.

In some embodiments, a device including the operational head includes an internal guiding element including one or more channels configured to provide dedicated channels to elements extending from the handle to the operational head.

In some embodiments, a system including the operational head includes an outer sheath configured to allow washing during the implantation procedure.

There is still further provided in accordance with some embodiments of the present disclosure, an implantation system for dilating a urethra at least partially obstructed by an enlarged prostate including:
a. a quasi-straight implant configured to be at least partially embedded through the wall of an enlarged portion of the prostate surrounding a constricted portion of the urethra;
b. a handle including a first motor configured to actuate linear movements, a second motor configured to actuate rotational movements and a controller configured to synchronize actuation between the first motor and the second motor;
c. an elongated body extending from the handle and connected to the first motor and configured to house an internal shaft including an operational head;
d. a shaft extending from the handle and connected to the second motor at its proximal end and including an operational head at its distal end.

In some embodiments, the operational head includes at least two arms connected to the shaft and including implant holders to which the quasi-straight implant is removably attached, the implant holders connected to the elongated body.

In some embodiments, the operational head includes a tissue cutter extending from the handle to the operational head and configured to perform a cut in the wall, the tissue cutter configured to rotate with the shaft.

In some embodiments, the operational head includes an expandable body surrounded by at least two arms and the quasi-straight implant is wrapped around the expandable element.

In some embodiments, the system further including an optical element extending from the handle inside the shaft and configured to allow a user to visualize the location of the constricted portion of the urethra.

In some embodiments, the implant is configured to be at least partially embedded through the wall of an enlarged portion of the prostate surrounding a constricted portion of the urethra.

There is still further provided in accordance with some embodiments of the present disclosure, an example method for dilating a urethra using an implant and a delivery system including an expandable body, the method including:
a. releasing a distal end of the implant
b. expanding the expandable body thereby expanding the urethra and;
c. releasing a proximal end of the implant.

In some embodiments, the method further includes loading the implant in a helix configuration on the delivery system.

In some embodiments, the method further includes inserting the delivery system into the urethra.

In some embodiments, the method further includes exposing a tissue cutter and rotating the delivery system so as to perform a cut in a wall of the urethra.

In some embodiments, the releasing the proximal end causes the implant to implanting the implant in the cut on the wall of the urethra.

In some embodiments, the implant is formed of a material, which expands outwardly in the cut of the wall of the urethra during and/or after implantation.

In some embodiments, the expanding the expandable body causes radially expanding the implant from the helix configuration to a C-shape arc configuration.

In some embodiments, the implant provides clearance between the enlarged prostate and a lumen of the urethra, and prevents recompression of the urethra due to further enlargement of the prostate.

In some embodiments, the method includes selecting the implant length and position to not interfere with an inner bladder valve.

There is still further provided in accordance with some embodiments of the present disclosure, an implantation system for dilating a urethra at least partially obstructed by an enlarged prostate including:
a. a quasi-straight implant configured to be at least partially embedded through the wall of an enlarged portion of the prostate surrounding a constricted portion of the urethra;
b. a delivery device for the quasi-straight implant including:
   i. a handle including at least one operational button and at least two motors;
   ii. an elongated body connected to the handle and configured to house an internal shaft including an operational head;
   iii. a shaft extending from the handle and interconnected to the at least two motors at its proximal end and including an operational head at its distal end;
   iv. an operational head including:
      A. at least two arms connected to the shaft and including implant holders to which the quasi-straight implant is removably attached;
      B. a tissue cutter extending from the handle to the operational head and configured to perform a cut in the wall, the cut being the place where the implant is to be at least partially embedded; and
      C. an expandable body surrounded by the at least two arms and the quasi-straight implant is wrapped around the expandable element;
c. an optical element extending from the handle inside the shaft and configured to allow a user to visualize the location of the constricted portion of the urethra.

There is still further provided in accordance with some embodiments of the present disclosure, an operational head for implantation system for dilating a urethra at least partially obstructed by an enlarged prostate including:
a. a quasi-straight implant configured to be at least partially embedded through the wall of an enlarged portion of the prostate surrounding a constricted portion of the urethra;
b. at least two arms connected to a shaft and including implant holders to which the quasi-straight implant is removably attached;
c. a tissue cutter extending from a handle to the operational head and configured to perform a cut in the wall, the cut being the place where the implant is to be at least partially embedded; and
d. an expandable body surrounded by the at least two arms and the quasi-straight implant is wrapped around the expandable element;
e. an optical element extending from the handle inside the shaft and configured to allow a user to visualize the location of the constricted portion of the urethra.

There is still further provided in accordance with some embodiments of the present disclosure, an operational head for implantation system for dilating a urethra at least partially obstructed by an enlarged prostate including:
a. a quasi-straight implant;
b. at least two arms connected to a shaft and including implant holders to which the quasi-straight implant is removably attached;
c. an expandable body surrounded by the at least two arms and the quasi-straight implant is wrapped around the expandable element;
d. an optical element extending from the handle inside the shaft and configured to allow a user to visualize the location of the constricted portion of the urethra.

There is still further provided in accordance with some embodiments of the present disclosure, an operational head for implantation system for dilating a urethra at least partially obstructed by an enlarged prostate including:
a. a quasi-straight implant;
b. at least two arms connected to a shaft and each arm including at least two implant holders to which the quasi-straight implant is removably attached;
the implant holders are configured to release the quasi-straight implant in a sequential manner so as to allow a controllable deployment of aid quasi-straight implant.

There is still further provided in accordance with some embodiments of the present disclosure, an implant for dilating a urethra at least partially obstructed by an enlarged prostate including:
a. a quasi-straight body;
b. pad-like endings at each end; and
c. one or more protrusions located at each end configured to stabilize the implant once implanted.

The present invention will be more fully understood from the following detailed description of embodiments thereof, taken together with the drawings, in which:

### BRIEF DESCRIPTION OF THE DRAWINGS

Figures 1a and 1b are schematic illustrations of generic views of a device for dilating a urethra of a subject at least partially obstructed by an enlarged prostate, according to some embodiments of the disclosure;
Figure 2 is a schematic illustration of a view of an exemplary elongated body of the device for dilating a urethra of a subject at least partially obstructed by an enlarged prostate, according to some embodiments of the disclosure;
Figure 3 is a schematic illustration of an exemplary embodiment of an internal operational element, according to some embodiments of the disclosure;
Figures 4a and 4b is a schematic illustration of an exemplary embodiment of an operational head in an inflated configuration, according to some embodiments of the disclosure;
Figure 5a is a schematic illustration of an exemplary expandable body, according to some embodiments of the disclosure;
Figure 5b is a schematic illustration of an exemplary jacket in an open configuration before it is mounted on an expandable body, according to some embodiments of the disclosure;
Figure 5c is a schematic illustration of an exemplary jacket in a close configuration after it is mounted on an expandable body, according to some embodiments of the disclosure;
Figure 6 is a schematic illustration of an exemplary implant holder/release mechanism located in the operational head, the operational head shown in a deflated configuration, according to some embodiments of the disclosure;
Figures 7a, 7b, 7c, 7d, and 7e are schematic illustrations of movement of a movable implant holder, according to some embodiments of the disclosure;
Figures 8a and 8b are schematic illustrations of exemplary tissue cutters positioned inside the shaft and outside the shaft, according to some embodiments of the disclosure;
Figures 8c, 8d, 8e, 8f, and 8g are schematic illustrations of the cutting mechanism using a rounded head cutter, according to some embodiments of the disclosure;
Figures 8h, 8i, 8j, 8k, and 8l are schematic illustrations of the cutting mechanism using a straight or "T-like" head cutter, according to some embodiments of the disclosure;
Figures 8m and 8n are schematic illustrations of the cutting angle, according to some embodiments of the disclosure;
Figures 8o and 8p are schematic illustrations of a direction of an exemplary cut in tissue performed according to some embodiments of the disclosure;
Figures 9a, 9b, and 9c are schematic illustrations of different views of an exemplary internal guiding element, according to some embodiments of the disclosure;
Figure 10 is a schematic illustration of an exploded view of an exemplary handle according to some embodiments of the disclosure;
Figures 11a and 11b are a schematic isometric view (Figure 11a) and an exploded view (Fig. 11b) of an exemplary internal actuation mechanism, according to some embodiments of the disclosure;
Figures 12a, 12b, 12c, and 12d are schematic illustrations of exemplary implants, according to some embodiments of the invention;
Figures 13a and 13b are schematic illustrations of additional exemplary hardware used with the device for dilating a urethra of a subject at least partially obstructed by an enlarged prostate, according to some embodiments of the disclosure;
Figures 14a and 14b are schematic illustrations of additional exemplary hardware used with the device for dilating a urethra of a subject at least partially obstructed by an enlarged prostate, according to some embodiments of the disclosure;
Figure 15 is a flowchart showing an exemplary method practiced according to some embodiments of the disclosure; and
Figure 16 is a flowchart showing an exemplary semi-automatic method practiced according to some embodiments of the disclosure.

### DETAILED DESCRIPTION

The present invention, in some embodiments thereof, relates to devices for treatment of intrabody lumens, and, more particularly, but not exclusively, to devices for dilating and/or assisting in dilation and/or maintaining dilation of the urethra to relieve obstruction resulting, for example, from benign prostatic hyperplasia (BPH). A broad aspect of some embodiments of the invention relates to increasing reliability in urethral implantation procedures by improving the device and the implant by means of improved engineering, interface and automation.

### Overview

Some embodiments of the disclosure relate to synchronizing actions to implant an implant in a cut made in the walls of the urethra. In some embodiments, one or more actions are performed by a device for dilating and/or assisting in dilation and/or maintaining dilation of the urethra to relieve obstruction resulting, for example, from benign prostatic hyperplasia (BPH). In some embodiments, one or more actions are performed by translating directional movements at a handle of the device to implantation actions at a distal end of the device. In some embodiments, directional movements are one or more of linear movements, or rotational movements, and any combination thereof. In some embodiments, movements at the handle are actuated by one or more motors controlled and/or synchronized by one or more controllers. In some embodiments, synchronization of actions includes one or more of synchronization of rotational movements with linear movement to correctly activate different parts of the device. In some embodiments, one or more actions are performed automatically one after another to allow repeatability of the implantation method and potentially reducing human error during implantation. In some embodiments, after each automated action(s) a visual and/or sonic alarm informs the user that the actions are finished.

In some embodiments, the device comprises a cutter that is configured to make the cut in the walls of the urethra and in tissue of the prostate in order to create an area shaped and sized to accommodate the implant. In some embodiments, the cut is created by the cutter such that the cut is wider deeper in the tissue compared to the cut at the surface of the tissue.

Some embodiments of the invention relate to an implant configured to be implanted inside the cut formed by the cutter in the walls of the urethra. Typically, the implant is configured to be at least partially embedded through the wall of an enlarged portion of the prostate surrounding a constricted portion of the urethra. In some embodiments, the implant is a thin implant configured to be implanted deeper in tissue when compared to less thin implants.

In some embodiments, the implant is made of a shape-memory alloy, such as nitinol. Typically, the shape-memory alloy of the implant is shape set such that the implant assumes a generally straight shape along more than 50 percent (e.g., more than 60 percent or more than 70 percent) of its length when in an unconstrained state (i.e., in the absence of any forces being applied to the implant). (In some instances, such an implant is described herein as a "quasi-straight implant".) Typically, upon implantation of the implant in the cut formed in the urethra, the implant is constrained into a curved shape by the tissue in which the implant is implanted. Due to the tendency of the shape-memory alloy to return to its pre-set generally straight configuration, the implant applies radially outward pressure to the tissue. By being shape set in the above-described manner, the implant typically dilates the urethra more effectively than if the implant were to be shape set such as to define a less generally straight shape.

In some embodiments, the implant comprises pads at the end of the implant configured to provide stability to the implant once implanted in the implantation site inside the cut. In some embodiments, the implant comprises one or more protrusions configured to stabilize the implant once implanted in the implantation site inside the cut, the protrusions shaped to be blunt so they will not damage the tissue.

Some embodiments of the disclosure relate to an implantation device configured to implant at least one implant in the urethra wall comprising at least one optical element, which allows the user to perform the implantation procedure under direct visualization of the desired implantation site and/or direct visualization of the implantation procedure itself and/or direct visualization of the correct implant implantation. In some embodiments, visualization of the implantation site is performed before inflating the inflatable body. In some embodiments, the optical element has a field of view of from about 0 degrees (meaning looking forward) to about ±90 degrees. In some embodiments, the desired implantation site is selected not to interfere with an inner bladder valve. In some embodiments, the desired implantation site is selected as the site which the urethra is most blocked. In some embodiments, visualization of the implantation procedure comprises one or more of visualizing the expansion of the urethra, visualizing the cutting of the wall of the urethra, visualizing the correct deployment of the implant, optionally inside the cut.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

In the following disclosure the term "distal" refers to the general direction farther from a user (e.g., a physician), while the term "proximal" refers to the general direction closer to the user; for example, something located distally may be in the body (e.g., towards the bladder), and proximal may be, for example, outside the body or towards a handle.

### Exemplary Generic View

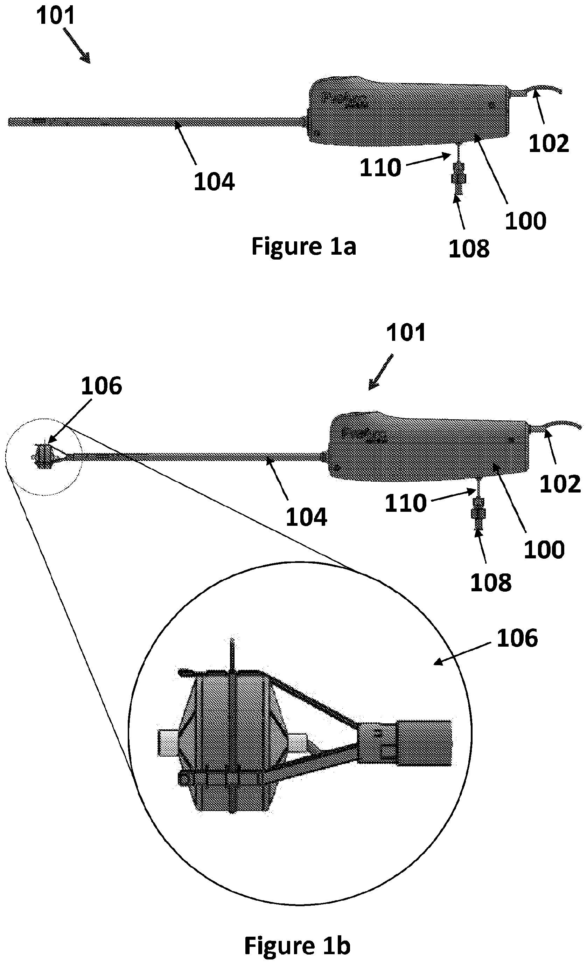

Referring now to the drawings, Figures 1a and 1b illustrates schematic generic views of the device for dilating a urethra of a subject at least partially obstructed by an enlarged prostate, according to some embodiments of the disclosure.

As shown, in some embodiments, the device comprises a delivery tool **101** having a proximal portion and a distal portion, the distal portion configured to be advanced to a location in an area of the urethra. In some embodiments, the device comprises a handle **100** configured to be held by a user. In some embodiments, the handle **100** is optionally connected to a power source (not shown) via a cable **102.** In some embodiments, the handle **100** optionally comprises a power source included within. In some embodiments, the delivery tool further comprises an elongated body **104** connected to the handle **100.** In some embodiments, the device further comprises an operational head **106**, as shown for example in Figure 1b, which is shown in an expanded configuration. In some embodiments, the device comprises an inlet/outlet **108**, connected to a tube **110** and configured to allow insertion and removal of fluids from the device, for example: air, water and/or saline.

### Exemplary Elongated body

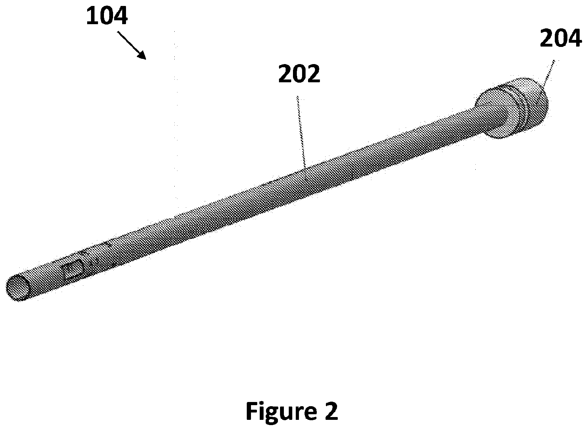

Referring now to Figure 2, showing a schematic view of an exemplary elongated body **104**, according to some embodiments of the disclosure.

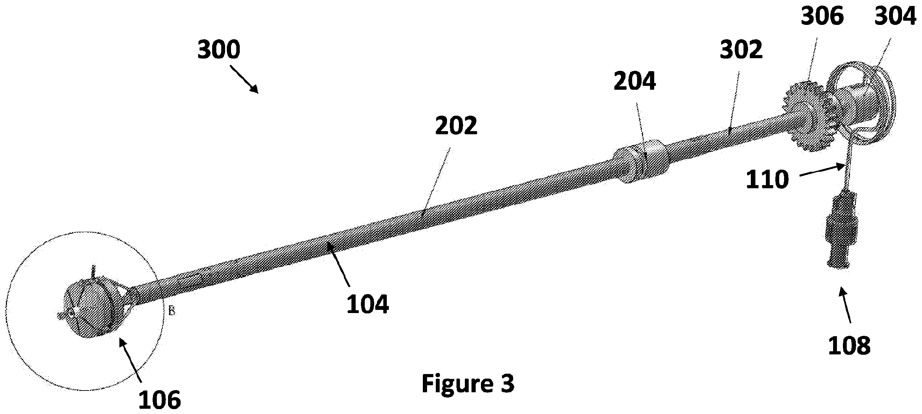

In some embodiments, the elongated body **104** comprises a tube **202** comprising a lumen sized and shaped to fit over an internal operational element **300** (shown in Figure 3), thereby providing a working channel for the internal operational element **300.** In some embodiments, elongated body **104** comprises a connector **204** at the proximal end configured to connect between the elongated body **104** and the handle **100** (shown in Figures 1a and 1b). In some embodiments, the internal diameter of the elongated body **104** is from about 3mm to about 20mm, optionally between 5mm and 15mm, optionally between 6mm and 10mm, for example, 5mm, 6mm, 7mm, 8mm, 10mm or 15mm. In some embodiments, the diameter of the elongated body **104** is calculated using the French catheter scale. In some embodiments, the diameter of the elongated body **104** is from about 5Fr to about 50Fr, optionally from about 10Fr to about 30Fr, optionally from about 15Fr to about 25Fr, for example 25Fr, 20Fr, 15Fr or 10Fr. In some embodiments, the elongated body **104** is made of, for example, metal or plastics, for example PEEK, PA, PEVAX, PTFE and/or FEP.

### Exemplary Internal Operational Element 300

Referring now to Figure 3, showing an exemplary embodiment of the internal operational element **300**, according to some embodiments of the disclosure.

In Figure 3, the elongated body **104** is also shown to provide reference. In some embodiments, the internal operational element **300** comprises a shaft **302** sized and shaped to be inserted inside the elongated body **104.**

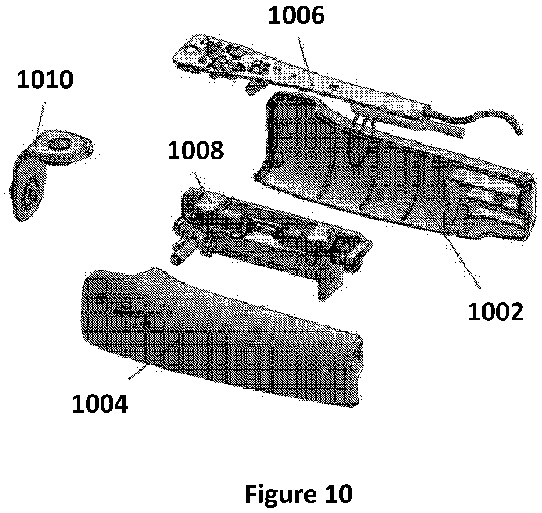

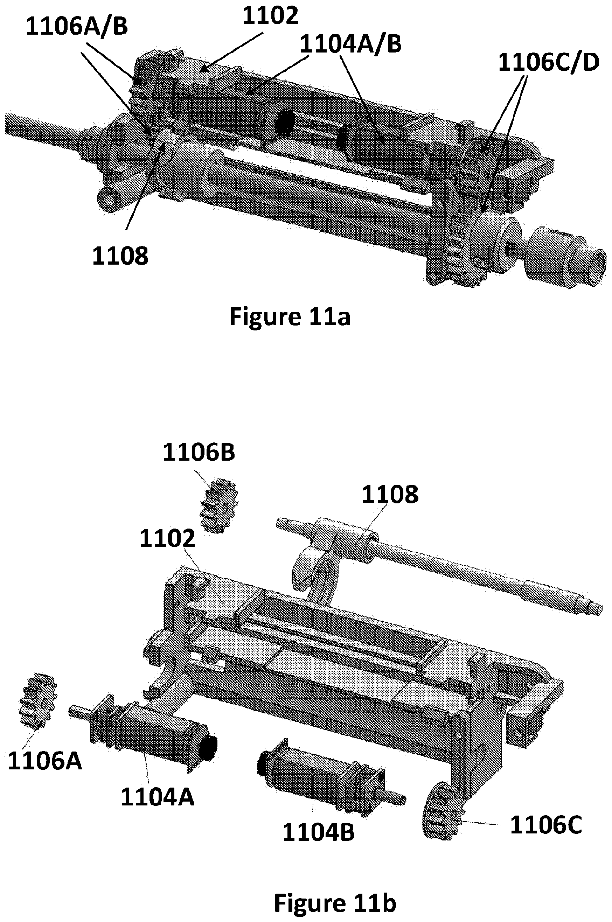

In some embodiments, at the proximal end of the internal operational element **300** there is a connector **304** configured to connect between the internal operational element **300** and the handle **100.** In some embodiments, the internal operational element **300** comprises a first gear **306** configured to interconnect between at least one motor **1104** (shown in Figures 11a-b) located at the handle and the operational head **106**, thereby providing an actuation mechanism for the operational head **106** (other gears and motors are disclosed below in the "*Exemplary handle and mechanisms therein*" section and in Figures 10 and 11a-b). In some embodiments, the internal diameter of the shaft **302** is from about 3mm to about 20mm, optionally between 5mm and 15mm, optionally between 6mm and 10mm, for example, 5mm, 6mm, 7mm, 8mm, 10mm or 15mm. In some embodiments, the diameter of the shaft **302** is calculated using the French catheter scale. In some embodiments, the diameter of the shaft **302** is from about 5Fr to about 50Fr, optionally from about 10Fr to about 30Fr, optionally from about 15Fr to about 25Fr, for example 25Fr, 20Fr, 15Fr or 10Fr. In some embodiments, the shaft **302** is made of, for example, metal or plastics, for example PEEK, PA, PEVAX, PTFE and/or FEP.

### Exemplary Operational Head

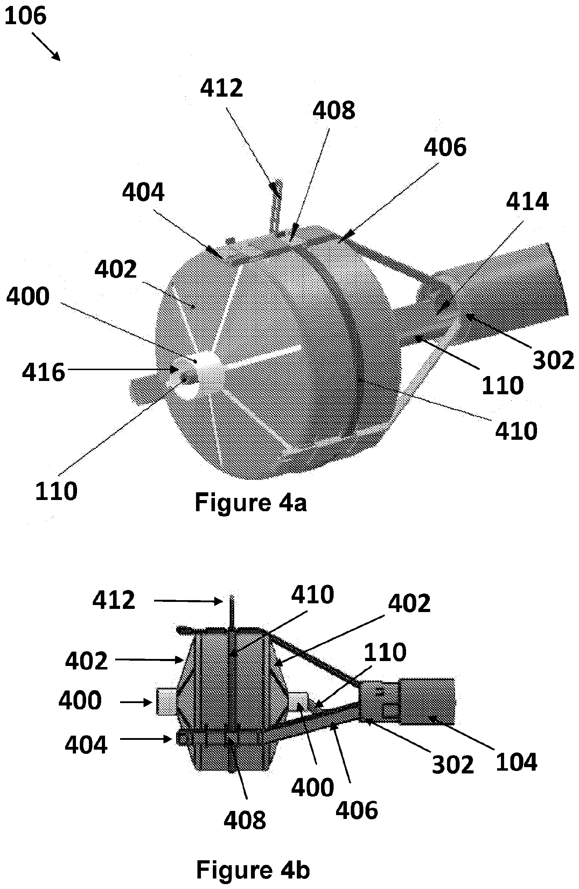

Referring now to Figures 4a and 4b, showing an exemplary embodiment of an operational head **106** in an expanded configuration, according to some embodiments of the disclosure.

In some embodiments, the operational head **106** comprises a plurality of elements configured to ensure the release of an implant at the chosen location. In some embodiments, the operational head **106** comprises an expandable body **400** (also referred to herein as an "expandable element"), one or more implant carrier arms **404** connected to the shaft **302** and comprising implant holding/release mechanisms, a tissue cutter **412**, an implant **410** and an internal guiding element **414.**

### Exemplary expandable body 400 and Jacket 402

Reference is still made to Figures 4a and 4b. In some embodiments, the operational head **106** comprises an expandable body **400** configured to be expanded and or retracted (inflated and/or deflated in case of a balloon) as chosen by the user. In some embodiments, the expandable body **400** is made of one or more materials, for example: PET, nylon, silicon, latex, polyurethane, Pebax^{®}. In some embodiments, the expandable body is made of super-elastic materials, for example nitinol. In some embodiments, the expandable body comprises an internal volume of from about 5mm³ to about 10mm³. Optionally from about 3mm³ to about 15mm³. Optionally from about 10mm³ to about 30mm³. In some embodiments, the diameter of the expandable body is from about 10mm to about 20mm. Optionally from about 5mm to about 35mm. Optionally from about 3mm to about 50mm. In some embodiments, the length of the expandable body is from about 5mm to about 80mm. Optionally from about 3mm to about 100mm. Optionally from about 20mm to about 150mm. In some embodiments, the expandable body **400** comprises a balloon. In some embodiments, the expandable body **400** is a balloon. 2. 1 atm = 101325 Pa. 2. In some embodiments, the expandable body can be inflated to an internal pressure of from about 1atm to about 20atm. Optionally from about 0.5atm to about 30atm. Optionally from about 5atm to about 50atm. In some embodiments, the expandable body **400** is in communication with the tube inlet/outlet **108.** In some embodiments, the expandable body **400** comprises a jacket **402** configured to enclose the expandable body **400.** In some embodiments, the jacket **402** is made of non-elastic materials, for example: PET and/or PA. In some embodiments, the jacket **402** provides the expandable body **400** with protection against damage and/or with a limit to the quantity of inflation that the expandable body **400** can be inflated. Figures 4a and 4b also shows the distal end of the tube **110**, which is connected to the expandable body **400.**

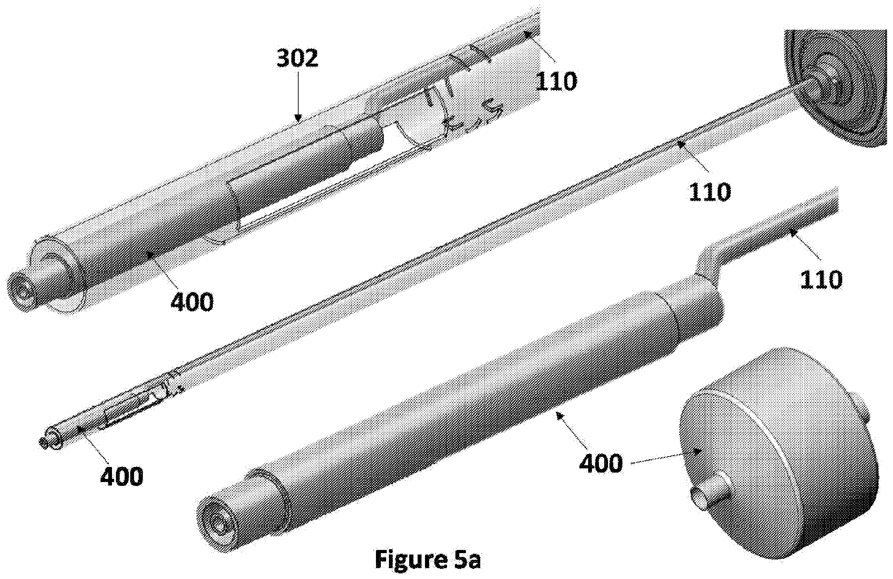

Referring now to Figure 5a, showing a schematic representation of an exemplary expandable body **400**, according to some embodiments of the disclosure.

Figure 5a shows the expandable body **400** in the deflated configuration connected to the tube. Also shown is the complex expandable body **400**/tube **110** with inside the shaft **302.** Also shown in Figure 5a is a schematic representation of the expandable body **400** in an inflated configuration. In some embodiments, the expandable body is manually inflated/deflated by the user. In some embodiments, the expandable body is automatically inflated/deflated by the device/controller.

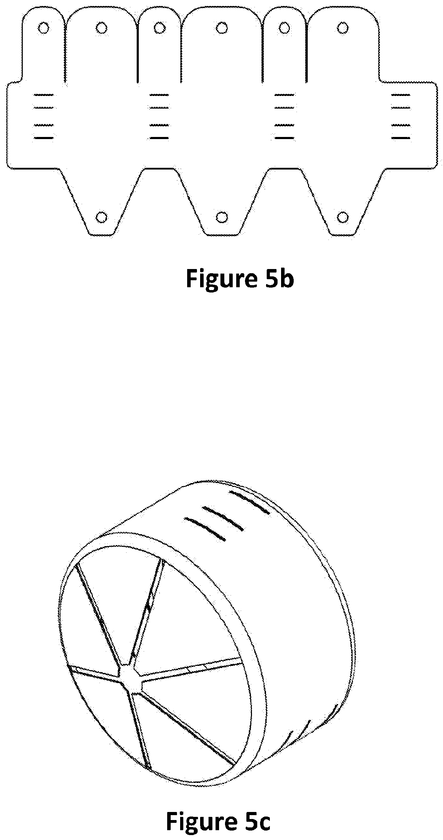

Referring now to Figures 5b and 5c: Figure 5b shows an exemplary jacket in an open configuration before it is mounted on the expandable body. Figure 5c shows an exemplary jacket alone as it would look like once mounted on the expandable body.

### Exemplary arms 404

Referring back to Figures 4a and 4b, in some embodiments, the operational head **106** further comprises one or more arms **404** connected to the shaft **302.** In some embodiments, the arms **404** are flexible arms. In some embodiments, the arms **404** are made of stainless steel or any other suitable metal. In some embodiments, there can be one arm, optionally two arms, optionally three arms, optionally four arms, optionally more. In some embodiments, a potential advantage of having three arms **404** is that it provides a good relationship between functionality and stability. In some embodiments, each arm **404** is covered by a sleeve **406.** In some embodiments, the sleeve **406** does not cover the entire length of the arm **404.** In some embodiments, the operational head **106** further comprises a movable implant holder **408** located between the arm **404** and the sleeve **406.** In some embodiments, the movable implant holder **408** is configured to hold the implant **410** in place.

In some embodiments, the order of the components is as follows, from internal to external: the expandable body **400** covered by the jacket **402.** The lower part of the sleeve **406** in contact with the jacket **402,** optionally attached to the jacket **402.** Inside the sleeve **406**, there is the arm **404.** On top of the arm **404** the implant **410** and on top on the implant **410** the movable implant holder **408.** In some embodiments, the implant **410** is held between the arm **404** and the movable implant holder **408**, which are held together by the sleeve **406.** Then, most external, the upper part of the sleeve **406.**

In some embodiments, the arms **404** are configured to perform inward-outward movements in relation to the longitudinal axis of the device. In some embodiments, the inward-outward movements accompany the inflation/deflation of the expandable body. In some embodiments, the inward-outward movements occur due to the inflation/deflation of the expandable body. In some embodiments, the arms **404** are configured to resist rotational movements in relation to the longitudinal axis of the device, when shaft **302** is rotated. In some embodiments, resistance to rotational movements is provided by the width of the arms **404.** In some embodiments, the arms **404** comprise a thickness of about 0.2mm, optionally from about 0.1mm to about 0.3mm, optionally from about 0.05mm to about 0.5mm. In some embodiments, the arms **404** comprise a width of about 2.5mm, optionally from about 2mm to about 3mm, optionally from about 1.5mm to about 5mm. In some embodiments, a relationship between the width of the arms and the thickness of the arms is of about 1/0.08, optionally of from about 1/0.01 to about 1/0.1, optionally from about 1/0.05 to about 0.5. In some embodiments, the arms **404** are made, for example, of stainless steel (for example, 302 stainless steel hard), and comprise an exemplary elastic modulus of about 200GPa ± 20% and a yield strength of about 1110MPa ± 20%.

### Exemplary Implant Holder/Release Mechanism

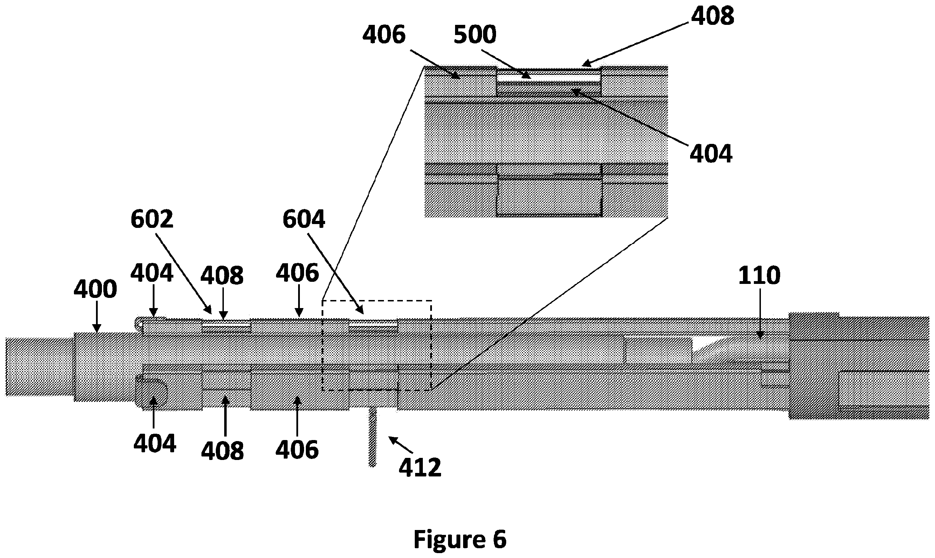

Referring now to Figure 6, showing an exemplary implant holder/release mechanism located in the operational head, the operational head shown in a deflated configuration, according to some embodiments of the disclosure.

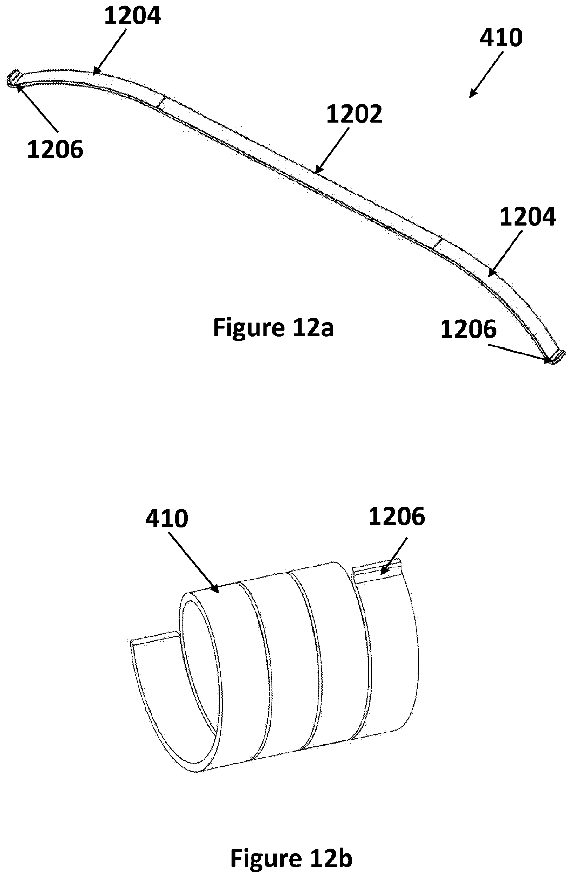

In some embodiments, as disclosed above, the operational head comprises arms **404** that extend over the expandable body **400**, as shown for example in Figure 6. In Figure 6, the expandable body **400** is in a deflated configuration (shown without the jacket **402**). Also, as disclosed above, each arm **404** comprises the sleeve **406** and the movable implant holder **408.** The zoom-in picture shows the space **500** created between the arm **404** and the movable implant holder **408.** In some embodiments, space **500** is configured to house the implant **410** (not shown). In some embodiments, each arm comprises one or more locations comprising implant holding locations. In some embodiments, some implant holding locations are located close to the distal end of the device, thereby named distal implant holders **602**, which are configured to hold a distal end and/or distal parts of the implant **410.** In some embodiments, some implant holding locations are located close to the proximal end of the device, thereby named proximal implant holders **604**, which are configured to hold a proximal end and/or proximal parts of the implant **410.** In some embodiments, since the implant is wrapped around the operational head **106**, as shown for example in Figure 12b, the distal end of the implant **410** is held by one of the distal implant holders **602**, then the implant is wrapped around the operational head so as to meet other implant holders until all implant holders comprise within them a part of the implant, and the proximal end of the implant is held by the last proximal implant holder **604.**

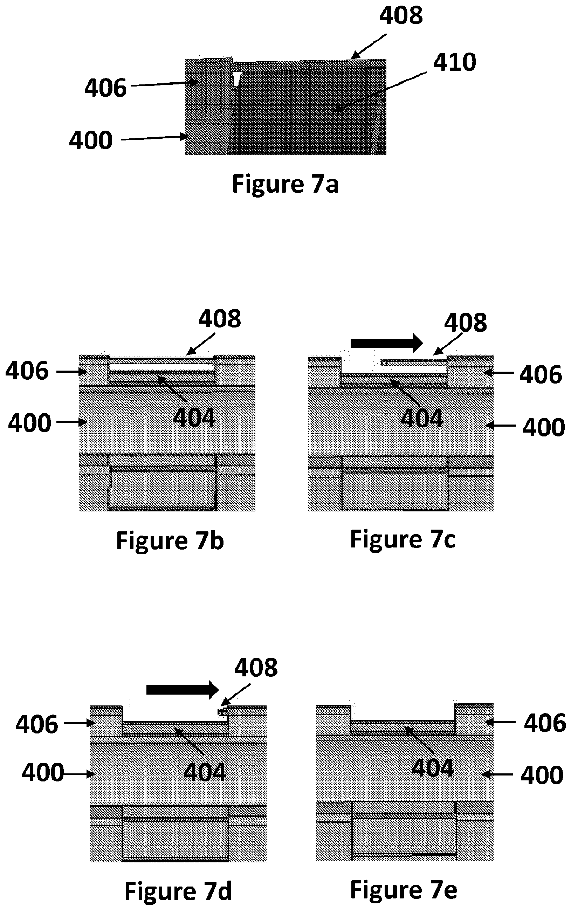

Referring now to Figures 7a-e, showing schematic illustrations of the movement of the movable implant holder **408,** according to some embodiments of the disclosure.

Figure 7a shows an exemplary implant **410** disposed between the movable implant holder **408** and the arm **404** (not shown because it is hidden by the implant), while held together by the sleeve **406.** Figures 7b-7e show an exemplary movement of the movable implant holder **408**, according to some embodiments of the invention, without the implant (just for clarity purposes). As can be seen in Figure 7b, the movable implant holder **408** is in its closed configuration, just before the beginning of the movement. Figure 7c shows, following the arrow, the movement of the movable implant holder **408.** Figure 7d shows, following the arrow, the movement of the movable implant holder **408** almost at the end of the movement. Figure 7e shows the movable implant holder **408** at its open configuration (movable implant holder **408** not actually shown since it is hidden by the sleeve **406**), thereby allowing the release of the implant **410.** In some embodiments, the proximal movement of the movable implant holder **408** causes firstly the release of the implant **410** from distal implant holders **602** and continuing the proximal movement will cause the release of the implant **410** from the proximal implant holders **604**, thereby providing a controllable and controlled release of the implant.

### Exemplary tissue cutter 412

Referring back to Figures 4a and 4b, in some embodiments, the operational head **106** comprises a tissue cutter **412** configured to cut the tissue of the urethra and create a space where the implant **410** will be implanted.

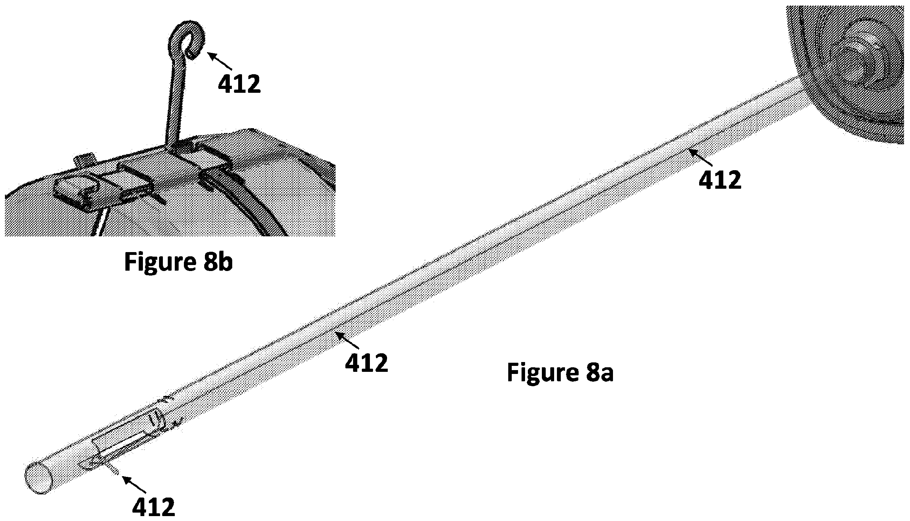

Referring now to Figures 8a-b, showing a schematic representations of exemplary tissue cutters **412** inside the shaft **302** (Figure 8a) and outside shaft **302** (Figure 8b), according to some embodiments of the disclosure.

In some embodiments, the tissue cutter **412** extends proximally at the handle and ends distally at the operational head of the device. In some embodiments, at the distal end, the tissue cutter **412** comprises a generally L-shaped operating configuration. In some embodiments, the tissue cutter **412** rotates (see below - methods) and the tissue cutter **412,** with the distal end of the generally L-shaped operating configuration, while in contact with the surface of the tissue performs a cut while rotating. In some embodiments, the tissue cutter **412** is delivered to the treatment area in a retracted configuration. In some embodiments, the tissue cutter **412** is then released and assumes its L-shaped operating configuration when actuated by the user. In some embodiments, once the tissue cutter **412** has performed its function, the tissue cutter **412** is retracted proximally, to avoid causing unwanted damage to the tissue. In some embodiments, the tissue cutter **412** is configured to perform a cut in the tissue having a depth of from about 2mm to about 10mm. Optionally from about 1mm to about 8mm. Optionally from about 4 to about 15mm. For example: 3mm, 5mm, 7mm, 10mm, 12mm. In some embodiments, the tissue cutter **412** is made of a resilient metal, for example, nitinol, or stainless steel. In some embodiments, the tissue cutter is connected to an electrical or electromechanical energy, for example a diathermy machine or a piezoelectric transducer. In some embodiments, cutting the tissue comprises using an electrified tissue cutter.

In some embodiments, the head of the tissue cutter **412** comprises a rounded end, as shown for example in Figure 8b, or a "T-like" shape (not shown), configured to provide a wider cut in a depth of the tissue while making a narrower cut on the surface of the tissue. In some embodiments, the length of the tissue cutter is from about 3mm to about 10mm. Optionally from about 5mm to about 8mm. Optionally from about 4mm to about 6mm. For example, 4mm, 5mm, 6mm, 7mm, 8mm.

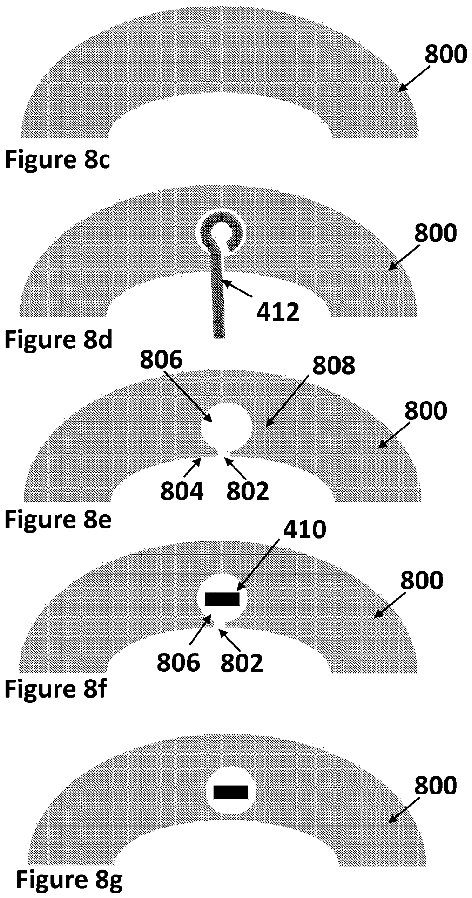

Referring now to Figures 8c-g, showing a schematic illustration of the cutting mechanism using a rounded head cutter, according to some embodiments of the disclosure.

Figure 8c shows a schematic illustration of the tissue **800**, in which the cut is performed. In some embodiments, the rounded head cutter **412** performs a cut in the tissue, as shown for example in Figure 8d. In some embodiments, due to the configuration of the cutter having a head comprises a rounded element, the cut that is done to define a narrow area **802** in the vicinity of the inner surface **804** (or optionally on the surface itself) of the tissue **800**, while making a wider cut **806** in the internal part **808** of the tissue **800**, as shown for example in Figure 8e. In some embodiments, the implant **410** is then inserted through the narrow area **802** into the wider area of cut **806** in the tissue **800**, as shown for example in Figure 8f. In some embodiments, since the cut performed on the surface of the tissue is narrow, this potentially enables a quicker healing of the surface, thereby closing the implant **410** inside the tissue, as shown for example in Figure 8g.

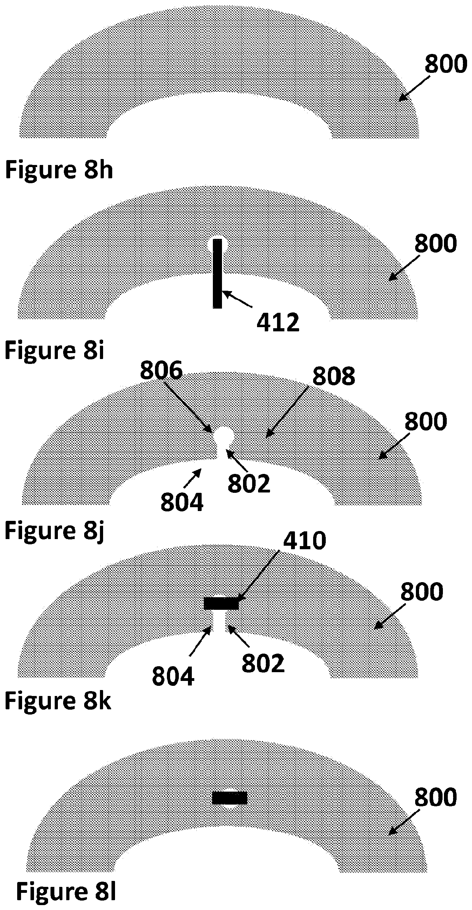

Referring now to Figures 8h-1, showing a schematic representation of the cutting mechanism using a straight or "T-like" head cutter, according to some embodiments of the disclosure.

Figure 8h shows a schematic representation of the tissue **800.** In some embodiments, the head cutter **412** performs a cut in the tissue, as shown for example in Figure 8i. In some embodiments, due to the configuration of the cutter having a straight head or a "T-like" head, the cut that is done comprises a narrow area **802** in the vicinity of the inner surface **804** (or optionally on the surface itself) of the tissue **800**, while optionally making a wider cut **806** in the internal part **808** of the tissue **800**, as shown for example in Figure 8j. In some embodiments, the implant **410** is then inserted through the narrow area **802** into the optionally wider cut **806** in the tissue **800**, as shown for example in Figure 8k. In some embodiments, since the cut performed on the surface of the tissue is narrow, this potentially enables a quicker healing of the surface, thereby closing the implant **410** inside the tissue, as shown for example in Figure 8l.

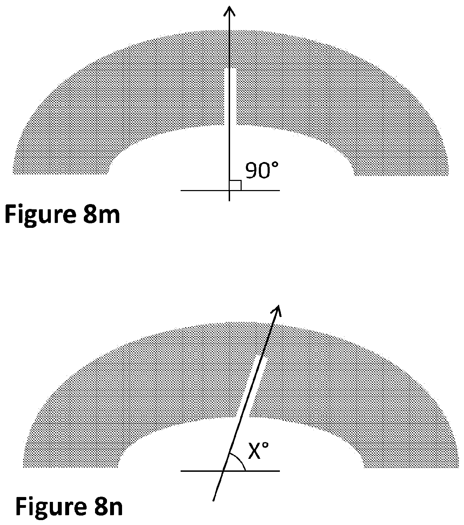

In some embodiments, the angle between the device and the cutter is 90 degrees, thereby when the cutter cuts the tissue, the cut is generally shaped to define a straight cut configuration, as schematically shown in Figure 8m. In some embodiments, the angle between the device and the cutter is other than 90 degrees, thereby when the cutter cuts the tissue, the cut is shaped to define an angled configuration having an angle X, as schematically shown in Figure 8n. In some embodiments, the angle is between about 45 degrees and about 89 degrees. Optionally between about 55 degrees and about 80 degrees. Optionally between about 60 degrees and about 75 degrees.

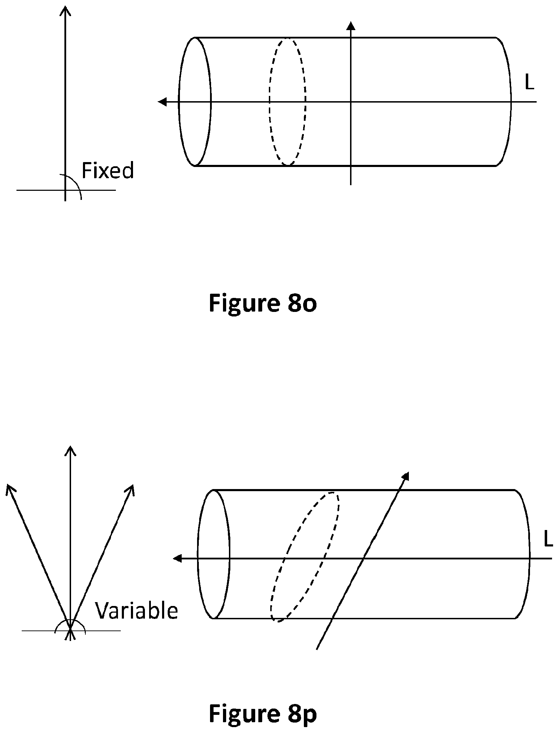

Referring now to Figures 8o-p, showing a schematic illustration of a direction of an exemplary cut, according to some embodiments of the disclosure.

In some embodiments, the angle between the device and the cutter stays the same during the cutting action, thereby providing a cutting having a straight direction, as shown for example in Figure 8o. In some embodiments, the angle between the device and the cutter varies during the cutting action, thereby providing a cutting having a non-straight direction in relation to the longitudinal axis **L** of the urethra, as shown for example in Figure 8p.

### Exemplary internal Guiding Element 114

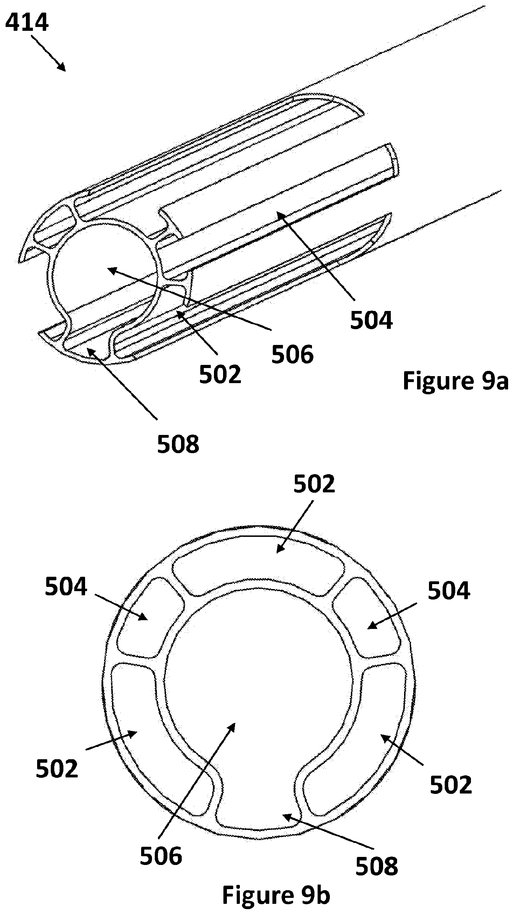

Referring now to Figures 9a-c, showing different view of an exemplary internal guiding element, according to some embodiments of the disclosure.

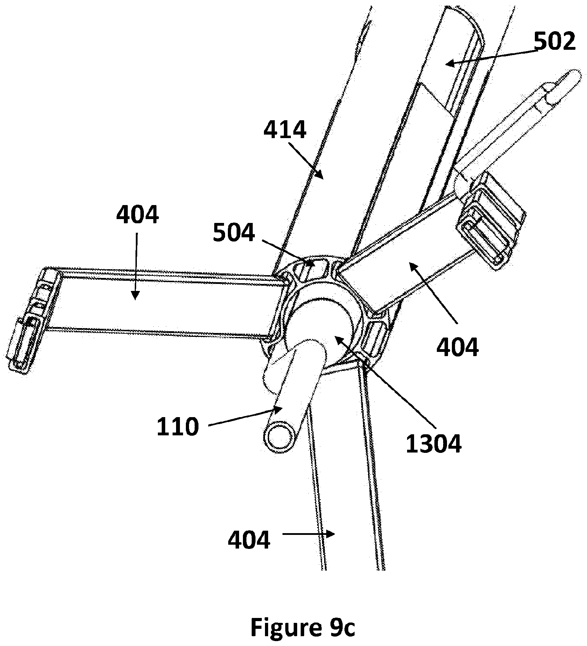

In some embodiments, inside the shaft **302** there can optionally be an internal guiding element **414** configured to separate between the different parts that run from the handle **100** to the distal end of the device. In some embodiments, the internal guiding element comprises one or more channels **502, 504, 506, 508** configured to house different parts, while optionally keeping them separated from each other, as shown for example in Figures 9a-b. In some embodiments, the internal guiding element **414** comprises an internal hollow channel **506.** In some embodiments, internal guiding element **414** comprises channels having different width, different depth, same width, same depth, and any combination thereof. In the exemplary internal guiding element **414** shown in Figures 9a-c, channels **502** are configured to house, in some embodiments, the arms **404**, optionally the arms **404** with the sleeve **406**, optionally the arms **404** with the movable implant holder **408**, optionally the arms **404** with the movable implant holder **408** with the sleeve **406.** In some embodiments, channels **502** also comprise the function to enable and assist with the rotational movement of the components located at the distal end, the rotational movement arriving from the handle to the distal end of the device. In some embodiments, channel **508** is configured to house the tube **110** connecting between the expandable body **400** and the inlet/outlet **108.** In some embodiments, channel **504** is configured to one or more of housing and isolating the tissue cutter **412.** In some embodiments, channel **506** is configured to house additional components used during the procedure, for example an optical element (e.g. micro camera - see below). Figure 9c shows a schematic image of the internal guiding element **414** with some of the different parts of the system.

### Exemplary handle and mechanisms therein

Referring now to Figure 10, showing an exploded view of a schematic illustration of an exemplary handle according to some embodiments of the disclosure.

In some embodiments, the handle **100** comprises dedicated covers **1002/1004** for the internal mechanisms in the handle **100**, the electronic hardware **1006**, the internal actuation elements **1008** and a frontal cover **1010** comprising the aperture for the shaft and the user control button(s). In some embodiments, the electronic hardware **1006** comprises a controller configured to synchronize between the actions performed by the device. In some embodiments, the actions of the device that are potentially synchronized includes one or more of activating motors to perform linear and/or rotational movements, inflating/deflating expandable body, exposing and/or retracting the cutter, exposing and/or retracting the operational distal end. In some embodiments, the controller is a mechanical controller. In some embodiments, the controller is an electronic controller.

### Exemplary internal actuation elements

Referring now to Figures 11a-b, showing a schematic isometric view (a) and exploded view (b) of an exemplary internal actuation mechanism, according to some embodiments of the disclosure.

In some embodiments, the internal actuation mechanisms are held by a chassis **1102** and one or more auxiliary/actuating rods/sleeves **1108.** In some embodiments, the internal actuation mechanisms comprise one or more motors **1104** configured to actuate mechanisms at the distal end of the device using of one or more gears **1106/306.** In some embodiments, the one or more motors **1104** are configured to rotate and/or actuate and/or move one or more of the elongated body **104**, the shaft **302**, the internal operational element **300**, the operational head **106**, the movable implant holders **408**, the tissue cutter **412**, and any combination thereof. In Figure 11a, the exemplary internal actuation mechanism is shown with the shaft **302**, as an example. In some embodiments, the one or more motors **1104** are the same. In some embodiments, the one or more motors **1104** are different. In some embodiments, the one or more motors **1104** are configured to operate at a velocity of from about 1RPM to about 1000RPM, optionally from about 1RPM to about 500RPM, optionally from about 1RPM to about 75RPM.

In some embodiments, the distal motor **1104A** rotates gear **1106A** which then rotates gear **1106B** which actuate the proximal/distal movement of the elongated body **104** (held by connector **204**) on the rod **1108.** 1 kgf = 9.81 N.

In some embodiments, the motor provides a force of from about 1Kg to about 5Kg. Optionally from about 2Kg to about 8Kg. Optionally from about 3Kg to about 10Kg. For example, 1Kg, 2Kg, 3Kg, 4Kg. In some embodiments, the motor provides a proximal-distal (or vice versa) movement at a velocity of from about 1mm/s to about 10mm/s. Optionally from about 2mm/s to about 20mm/s. Optionally from about 4mm/s to about 30mm/s. For example, 2mm/s, 4mm/s, 8mm/s, 15mm/s.

In some embodiments, the proximal motor **1104B** rotates gears **1106C** and **1106D** (also numbered gear **306** in Figure 3) which actuate rotational movement of the shaft **302.** In some embodiments, the motor **1104B** is configured to provide shaft **302** with a rotational force (torque) of from about 50Nmm to about 300Nmm. Optionally from about 80Nmm to about 500Mnn. For example, 90Nmm, 100Nmm, 112Nmm, 120Nmm. In some embodiments, the velocity of rotation of the shaft **302** is of from about 1 rotation per second to about 1 rotation per 3 seconds. Optionally from about 1 rotation per 0.5 second to about 1 rotation per 5 seconds. For example, 1 rotation in 1 second, 1 rotation in two seconds, 1 rotation in 3 seconds.

### Exemplary Implants

Referring now to Figures 12a-d, showing exemplary implants **410**, according to some embodiments of the invention. Figure 12a shows an exemplary implant **410** in an open configuration, while Figure 12b shows an exemplary implant in a folded spiral configuration. In some embodiments, the implant **410** is made of a suitable resilient material, for example one or more of stainless steel, nitinol, titanium, PET, PEEK, PA. In some embodiments, the implant **410** comprises a length of from about 50mm to about 70mm. Optionally from about 40mm to about 80mm. Optionally from about 30mm to about 100mm. For example, 50mm, 60mm, 70mm, 80mm. In some embodiments, the implant **410** comprises a thickness of from about 0.01mm to about 1mm, optionally from about 0.1mm to about 0.8mm, optionally from about 0.3 to about 0.6mm, for example 0.1mm, 0.25mm, 0.3mm, 0.5mm. In some embodiments, the implant **410** comprises a width of from about 0.5mm to about 4mm, optionally from about 0.7mm to about 2mm, optionally from about 1mm to about 1.5mm, for example. 0.5mm, 0.7mm, 1mm, 1.3mm, 2mm, 3mm. In some embodiments, a potential advantage of making implants having a short width is that implants having a short width allow the tissue around the implant to close and heal better, and they allow the implants to be inserted deeper in the cut.

In some embodiments, the implant **410** is manufactured straight. In some embodiments, the implant **410** is manufactured slightly curved. In some embodiments, the implant **410** comprises a middle straight section **1202** and two slightly curved end sections **1204** (e.g., end sections 1204 are also referred to herein as the proximal and distal portions of the implant), as shown for example in Figure 12a. It is noted that for some embodiments, end sections **1204** are shaped to define straight end sections/portions when implant 410 is unconstrained. In some embodiments, at the end sections of the implant **410**, the implant is folded thereby creating protrusions **1206**, as shown for example in Figures 12a-b. In some embodiments, the folded portion (protrusion) is used to hold the implant between the arm and the movable implant holder **408.** In some embodiments, the foldings (protrusions) **1206** at the end of the implant provide further anchoring elements once inside the cut. In some embodiments, the shape of the implant is configured to provide the implant with an increased range of the forces that the implant applies on the tissue wall. In some embodiments, the forces applied by the implant to the walls of the urethra are of from about 50gr to about 100gr. Optionally from about 40gr to about 150gr. Optionally from about 25gr to about 500gr. For example 50gr, 60gr, 70gr, 100gr, 120gr.

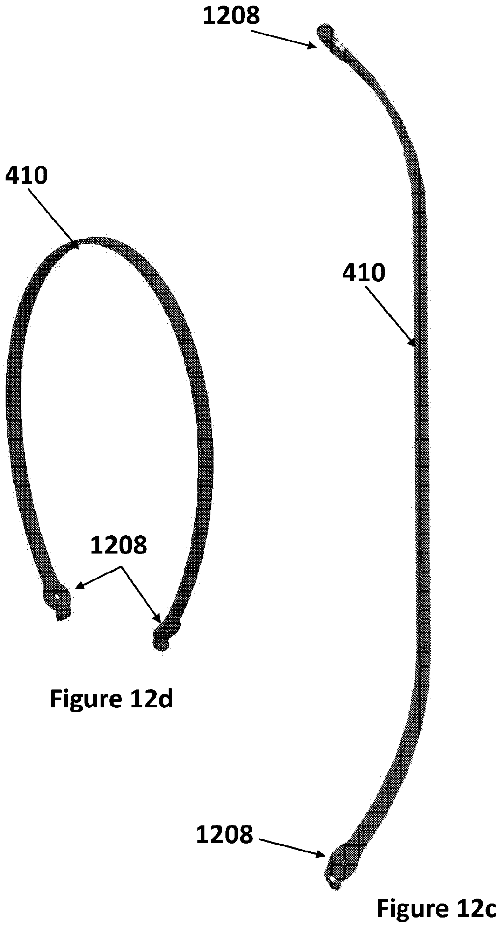

Typically, implant 410 is made of a shape-memory alloy, such as nitinol. Typically, the shape-memory alloy of the implant is shape set such that the implant assumes a generally straight shape at least along middle section 1202 when in an unconstrained state (i.e., in the absence of any forces being applied to implant 410). Since the implant 410 is made of shape-memory materials that tend to return to their natural open state, once released from the delivery device, the implant will try to return to its shape set, open state, which is substantially straight, thereby pushing the walls of the urethra with an increase force, when compared to an implant that is not substantially straight. In some embodiments, the inflation of the expandable body helps the implant to return to the desired configuration. Figures 12a and 12c show exemplary implants in their natural open state having a generally straight configuration. Figure 12d shows the form that the implant will take inside the cut on the wall of the urethra once implanted. As shown in Figure 12d, implant 410 is implanted in the tissue in a curved configuration. In some embodiments, due to the resistance created by the walls of the urethra, the implant is not be able to return to its straight configuration, thereby continuing pushing the walls and thereby opening the obstruction of the urethra.

Typically, the shape-memory material of the implant is shape set such that the implant assumes a generally straight shape along more than 50 percent (e.g., more than 60 percent or more than 70 percent) of its length when in an unconstrained state (i.e., in the absence of any forces being applied to the implant). (In some instances, such an implant is described herein as a "quasi-straight implant".) Typically, upon implantation of the implant in the cut formed in the urethra, the implant is constrained into a curved shape by the tissue in which the implant is implanted. Due to the tendency of the shape-memory alloy to return to its pre-set generally straight configuration, the implant applies radially outward pressure to the tissue. By being shape set in the above-described manner, the implant typically dilates the urethra more effectively than if the implant were to be shape set such as to define a less generally straight shape.

In some embodiments, the implant comprises pad-like endings **1208**, as shown for example in Figures 12c-d. In some embodiments, the pad-like endings **1208** contribute to one or more of: the correct placement of the implant inside the cut by not allowing the implant to rotate on its axis once inside the cut and/or potentially avoiding perforating and/or damaging the tissue once inside the cut.

In some embodiments, the implant comprises one or more protrusions configured to stabilize the implant once implanted in the implantation site inside the cut, the protrusions configured to be blunt so they will not damage the tissue.

In some embodiments, the implant is removed after a predetermined period of time, for example 3 months, 6 months, 12 months or 36 months. Alternatively, the implants are formed of a material that is biodegradable. In some embodiments, the implants biodegrade over a period of time chosen by the doctor, for example 3 months, 6 months, 12 months or 36 months.

### Exemplary additional hardware

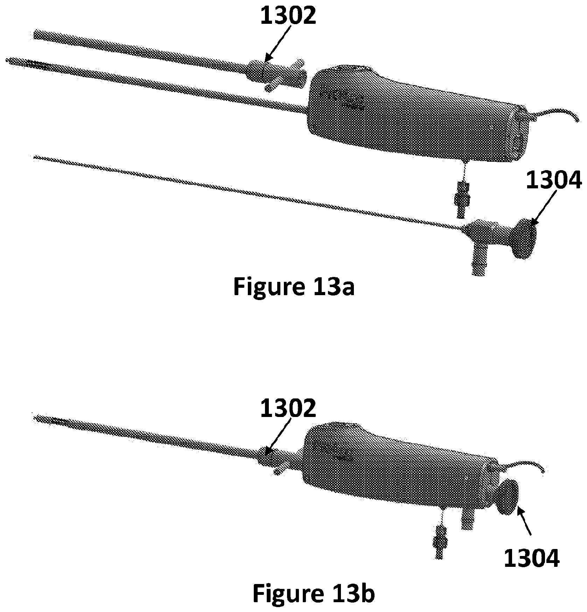

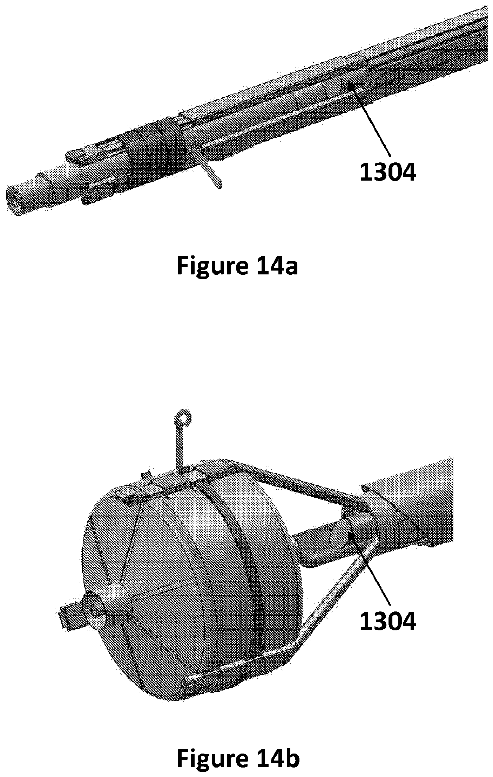

Referring now to Figures 13a-b and 14a-b, showing two exemplary additional hardware used with the device for dilating a urethra of a subject at least partially obstructed by an enlarged prostate, according to some embodiments of the disclosure.

In some embodiments, the device is used together with an outer sheath **1302.** In some embodiments, the device further comprises an optical element **1304.** Figure 13a shows the device separated from the additional hardware of outer sheath **1302** and optical element **1304**, while Figure 13b shows the device with the additional hardware outer sheath **1302** and optical element **1304** incorporated therein.

### Exemplary outer sheath (Obturator)

In some embodiments, an outer sheath (obturator) **1302** is used during the treatment, as known in the art. In some embodiments, the outer sheath (obturator) **1302** is sized and shaped to house the device.

### Exemplary optical element (camera)

Referring now to Figures 14a-b, showing the distal end of an exemplary optical element, for example a camera, at the distal end of the device, according to some embodiments of the disclosure.

In some embodiments, the device further comprises the optical element **1304**, which extends from the proximal end of the device until slightly beyond the end of the shaft, about ±2mm of the end of the shaft. In some embodiments, the optical element extends until before the expandable body. In some embodiments, the optical element comprises a field of view of from about 0 degrees (meaning looking forward) to about ±90 degrees, optionally from about 0 degrees to about ±70 degrees, optionally from about 0 degrees to about ±120 degrees. In some embodiments, the optical element is configured to allow the user to navigate through the urethra, visualize the location of the prostate, and place the implant under direct visualization at and of the desired place.

### Exemplary Methods

### Exemplary manual method

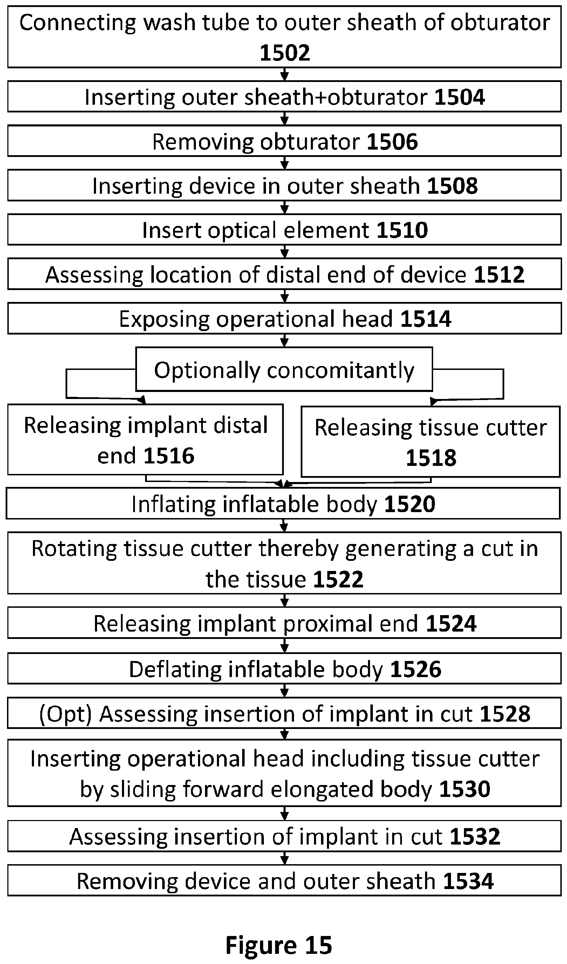

Referring now to Figure 15 showing a flowchart disclosing an exemplary method practiced, according to some embodiments of the disclosure.

In some embodiments, the following method is followed by a user during the operation of the device. In some embodiments, the method begins by connecting a wash tube to the outer sheath of an obturator **1502.** Then, the user inserts the outer sheath with the obturator in the patient **1504.** Then the user removes the obturator, leaving the outer sheath in place **1506.** Then the user inserts the device in the outer sheath **1508.** In some embodiments, optionally, the user then inserts the optical element (e.g., a camera) inside the device **1510.** In some embodiments, the user then assesses the location of the distal end of the device by means of the optical element (camera) **1512.** In some embodiments, the operational head is then exposed beyond the distal end of the device **1514** by retracting (moving proximally) the elongated body **104** from about 30mm to about 40mm, preferably 36mm. In some embodiments, then the distal end of the implant is released from device **1514** by further retracting (moving proximally) the elongated body **104** from about 3mm to about 7mm, preferably 5mm. In some embodiments, releasing the distal end of the implant allows the expandable body to be inflated, since if both ends of the implant are trapped, the implant will hold the arms therefore not permitting the arms to extend providing the expandable body with the necessary space to be inflated. In some embodiments, then the tissue cutter 1518 is then released. In some embodiments, up to this point, the tissue cutter is held inside the outer tube of the device. In some embodiments, only when cutting is required, the tissue cutter is released from the outer tube. In some embodiments, optionally, the releasing of the implant distal end **1516** and the releasing of the tissue cutter **1518** are performed concomitantly. In some embodiments, then the expandable body is inflated **1520.** In some embodiments, the inflation of the expandable body extends the recently one side released implant, from its folded confirmation to its expanded configuration. In some embodiments, then the tissue cutter is rotated thereby generating a cut in the tissue **1522.** In some embodiments, rotation of the cutter is performed by rotating the outer tube together with all the components inside of it. In some embodiments, the following action is the release of the proximal end of the implant **1524** by further retracting (moving proximally) the elongated body **104** from about 5mm to about 9mm, preferably 7mm.

In some embodiments, the release of the proximal end of the implant will allow the implant to unwind from the folded helical configuration (the folded configuration shown in Figure 12b) into a curved configuration in which it is released and implanted into the cut (the curved configuration as shown in Figure 12d). Since the release of the implant is performed in vicinity to the cut, the implant enters the cut. In some embodiments, then the expandable body is deflated **1526.** In some embodiments, optionally at this point the user can assess the insertion of the implant in the cut **1528.** In some embodiments, then the operational head is inserted back into the elongated body for subsequent removal from the body by sliding forward the elongated body, optionally together with the tissue cutter **1530.** In some embodiments, optionally the user then assesses (again if done before or for the first time if not performed before) the deployment of the implant in the cut **1532.** In some embodiments, the method ends by removing the device with the outer sheath from the body of the patient **1534.**

### Exemplary semi-automatic method

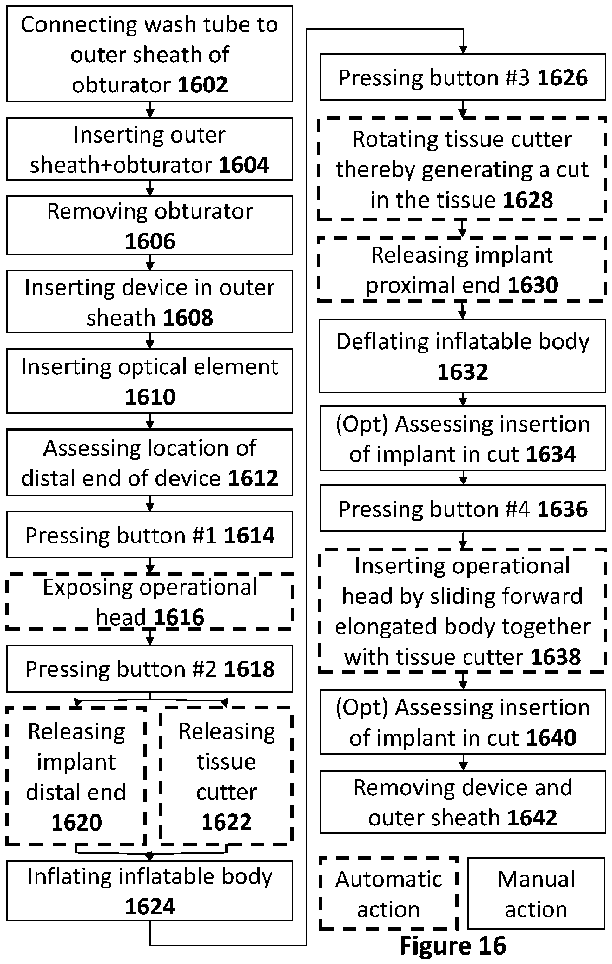

Referring now to Figure 16 showing a flowchart disclosing an exemplary semi-automatic method practiced, according to some embodiments of the disclosure.

In some embodiments, the following semi-automatic method is followed by a user during the operation of the device. In the flowchart solid squares are manual actions, while dashed squares are automatic actions activated by the user (for example, by pushing a button). In some embodiments, the method begins by connecting a wash tube to the outer sheath of an obturator **1602.** Then, the user inserts the outer sheath with the obturator in the patient **1604.** Then the user removes the obturator, leaving the outer sheath in place **1606.** Then the user inserts the device in the outer sheath **1608.** In some embodiments, optionally, the user then inserts the optical element (e.g., a camera) inside the device **1610.** In some embodiments, the user then assesses the location of the distal end of the device by means of the optical element (camera) **1612.** In some embodiments, the user presses the button for the first time **1614**, which causes the operational head to automatically be exposed beyond the distal end of the device **1616** by retracting (moving proximally) the elongated body **104** from about 30mm to about 40mm, preferably 36mm. In some embodiments, the user then presses the button for the second time **1618**, which causes the automatic release of the distal end of the implant **1620** by further retracting (moving proximally) the elongated body **104** from about 3mm to about 7mm, preferably 5mm. In some embodiments, releasing the distal end of the implant allows the expandable body to be inflated, since if both ends of the implant are trapped, the implant will hold the arms therefore not permitting the arms to extend providing the expandable body with the necessary space to be inflated. In some embodiments, optionally, concomitantly with the release of the distal end of the implant **1620**, the tissue cutter is automatically released **1622.** In some embodiments, then the inflatable body is manually inflated **1624.** In some embodiments, the inflation of the expandable body extends the recently one side released implant, from its folded confirmation to its expanded configuration. In some embodiments, the user then presses the button for the third time **1626**, which causes the automatic rotation of the tissue cutter thereby generating a cut in the tissue **1628.** In some embodiments, rotation of the cutter is performed by rotating the outer tube together with all the components inside of it.

In some embodiments, following the automatic rotation **1628**, there is the automatic release of the proximal end of the implant **1630** by further retracting (moving proximally) the elongated body **104** from about 5mm to about 9mm, preferably 7mm. In some embodiments, the release of the proximal end of the implant will allow the implant to unwind from the folded helical configuration (the folded configuration shown in Figure 12b) into a curved configuration in which it is released and implanted into the cut (the curved configuration as shown in Figure 12d). Since the release of the implant is performed in vicinity to the cut, the implant enters the cut. In some embodiments, the user then manually deflates the expandable body **1632.** In some embodiments, optionally the user then assesses the insertion of the implant in the cut **1634.** In some embodiments, the user then presses the button for the fourth time **1636,** which causes the automatic insertion of the operational head in the outer tube optionally together with the tissue cutter **1638.** In some embodiments, optionally the user then assesses (again if done before or for the first time if not performed before) deployment of the implant in the cut **1640.** In some embodiments, the method ends by removing the device with the outer sheath from the patient **1642.**

In some embodiments, valid to any of the methods disclosed herein, the rotational movement of the device, which performs the cutting, is concomitantly performed with the gradual release of the implant into the cut, therefore, at the end of the rotation the implant is completely released.

### Exemplary automatic method controlled by controller

In some embodiments, the user (operator) controls the movement of the mechanisms by pressing on a single button that activates the controller to perform a synchronized step-by-step implant release method. In some embodiments, as mentioned above, the delivery system comprises of two controlled motors: a linear motor - moving the outer sheath (left to right and right to left, as explained above), and a rotary motor - moving the shaft assembly in a clockwise direction to create the circumferential cut inside the tissue. In some embodiments, the software in the controller also comprises instructions to control and synchronize the motor movement with the cutter.

The following Table A discloses an exemplary relationship between the actions of the linear motor, the rotary motor, the parts of the device and the action of the implanting method. The following Table A does not mean to be limiting and it is brought to allow a person having skills in the art to understand the invention.

**Table A**

| Stage | Linear motor | Rotary motor | Part that moves | Action performed | Method action |
|---|---|---|---|---|---|
| User presses button | | | | | |
| 1 | On | Off | Elongated body **104** | Moves backwards (proximally) | Exposing operational head |
| 2 | On | Off | Elongated body **104** | Moves backwards (proximally) | Releasing implant distal end |

| User inflates expandable body and presses button - or optionally inflation is performed automatically. | | | | | |
|---|---|---|---|---|---|
| 3 | Off | On | Shaft **302** rotates and cutter with it | Clockwise rotation | Cutting tissue |
| 4 | On | Off | Elongated body **104** | Moves backwards (proximally) | Releasing implant proximal end |

| Optionally, the system stops to allow user to assess correct insertion of implant and then user presses button | | | | | |
|---|---|---|---|---|---|
| User deflates expandable body - or optionally deflation is performed automatically. | | | | | |
| 5 | On | Off | Elongated body **104** | Moves forward (distally) | Covering operational head |
| Optionally, device disables use of button | | | | | |

### Exemplary emergency stop button

In some embodiments, the device comprises at least one emergency button configured to stop the action that the device is performing at that moment. In some embodiments, pressing the emergency button once stops the action that is performed in that moment. In some embodiments, pressing the emergency button twice causes the device to completely retract inside the shaft, including when relevant but not exclusively, deflating the expandable body, retracting the cutter, rotating to insert the implant inside the shaft.

### Exemplary visual indications to the user

In some embodiments, after each time that the user presses a button, a visual indication is shown to the user to let him know that the automatic actions are finished and that the next step in the method can be performed. In some embodiments, visual indications can be led light of a variety of colors. In some embodiments, visual indications can be a digital screen.

### Exemplary non-urethral use

In some embodiments, the device and methods of implantation are used for other parts of the body that require opening of a tubular member or any other body lumen, for example the digestion system, the vascular system, etc.

Various embodiments and aspects of the present invention as delineated hereinabove and as claimed in the claims section below find support in the following examples.

### Example of operating instructions for use of the device

For some embodiments, the apparatus described herein is used in conjunction with one or more of the following pieces of equipment:
· 380mm, 2.9 mm, 12° telescope (CL-SCOPE)
· 24F sheath (CL-SHEATH)
· Visual Obturator (CL-VO)
· Cystoscopy camera, light box/cable and monitor
· Olympus ESG-400 Universal HF generator
· Standard fluid irrigation system including new, sterile fluid tubing
· Standard endoscopic grasper kit

For some such embodiments, the operating instructions for use of the apparatus include one or more of the following steps. It is noted that these steps are provided purely by way of example, and that some or all of these steps are not necessarily implemented.

### 1. PRE-PROCEDURE CYSTOSCOPY

**1.1** Assemble the 2.9 mm 12° telescope (CL-SCOPE), visual obturator (CL-VO), and 24F (CL-SHEATH).
**1.2** Advance the telescope assembly through the urethra and visualize both the urethra and bladder by advancing into the bladder.
**1.3** Remove the telescope and visual obturator, leaving the sheath in the bladder.

### 2. PREPARATION

**2.1** Confirm that packaging components are unopened and undamaged.
**2.2** Inspect all components for any damage that may have occurred during shipment or other handling.
**2.3** While holding the handle end (heavy end) of pouch, open the pouch by peeling the sides (device handle end) to access the sterile contents.
**2.4** Using sterile technique, remove the plastic tray out of the pouch.
**2.5** Using sterile technique, remove the device from the packaging by grasping the handle and pulling the device out of the tray the tray.
**2.6** Inspect the device tip and confirm that the distal balloon is not visible.
**2.7** Connect the Delivery System to the auxiliary equipment as follows:
   1. Insert 2.9 mm 12° telescope (CL-SCOPE) into device with the telescope lightpost at 6 o'clock. Keep forward pressure on the telescope, hold telescope lightpost at 6 o'clock, and secure the scope by pressing the scope in until a "click" is felt.
   2. Connect the cystoscope camera on the telescope and light source to the lightpost connector
   3. Connect the power cable to the power supply
   4. Connect the blue electrode connector to the Olympus ESG-400 Universal HF generator and set to BLENDCUT 50 setting
   5. Insert the delivery system through the outer sheath (CL-SHEATH)

### 3. DEVICE INSERTION AND POSITIONING

**3.1** Distal Tip partial exposure by pressing the button (Step 1 - 1^{st} button press), Dilatation Balloon Exposure for increase field of view.
**3.2** Locate the treatment site by visualizing the prostatic fossa from the bladder neck to the verumontanum.
**3.3** Partial Implant Release by pressing the button (Step 2 - 2^{nd} button press), initial deploying of the implant and cutting element exposure. The flexible Nitinol implant extends 33 mm from the side of the distal of the dilatation balloon.
**3.4** To avoid external prostatic structures (e.g., neurovascular bundles), position the Delivery Device tip in the anterior aspect of the prostate in either the 2-3 or 9-10 o'clock position. Orient the tip to 12 o'clock to ensure the implant deploys the center between the two lateral lobes (implant deploys around the dilatation balloon.
   As with cystoscopy, keep device parallel to the prostatic fossa and avoid excessive instrument movement throughout positioning and deployment.
   To obtain the desired urethral opening, place implants throughout the length of the lateral prostate lobes at approximately 1 cm to 1.5 cm intervals starting 1.5 cm distal to the bladder neck.
**3.5** Position the Delivery Device such that the Marker is against the target prostatic middle section lobe in the lateral upward direction.
**3.6** To achieve desired amount of urethral opening, angle the Delivery Device to 6 o'clock and push the distal end upward, applying slight pressure to the Delivery Device tip via Delivery Device handle.

### 4. IMPLANT DEPLOYMENT

While holding the Delivery Device distal tip stable against the target tissue, perform the following steps:
**4.1** Inflate the dilatation balloon with the pressure control syringe (Figures 4a and 4b) up to 2atm. Make sure dilatation balloon is fully inflated by visualization of the inflated balloon on the screen monitor.
**4.2** Trigger Urethral Implant Release sequence by pressing the button (Step 3 - 3^{rd} button press), creating a circumferential urethral cut inside the prostate and deploying nitinol implant inside the created cut. The System circumferential urethral cut is up to 5 mm deep inside the prostate gland tissue which is sufficient to reliably position the prostatic implant inside the cut based on cadaver and clinical studies.
**4.3** After the implant is fully deployed, hold still for up to 10 seconds, deflate the dilatation balloon by creating a vacuum pressure until syringe is filled up to 40cc while holding it upward.
**4.4** To withdraw the delivery device and press the button (Step 4 - 4^{th} button press) to cover the distal end assembly. The Delivery Device can then be removed from the cystoscopy sheath. Gently pull the delivery device with light force in back-and-forth movements. As with cystoscopy, keep the device parallel to prostatic fossa. When advancing the Delivery Device distally, maintain the Delivery Handle and in the center between the lobes.
**4.5** To deploy an additional implant, remove Delivery Device from the sheath and replace with a new System.
   To obtain the desired urethral opening, place implants throughout the length of the urethral prostate at approximately 1 cm intervals.
**4.6** Cystoscopy may be performed in between and/or after implant deployments to cystoscopically address if any additional implants are needed. If an additional implant is needed, see section 4, Delivery Device Positioning: Lateral Lobe. If no obstruction persists, continue to section 6, Cystoscopy.

### 5. CYSTOSCOPY

Between and after implant deployments, perform a cystoscopy of the urethra and bladder to:
- Confirm the desired effect has been achieved.
- Confirm that all implant components are well embedded inside the mucosal tissue within the prostatic urethra.
- Ensure implants are not present in the bladder or at the bladder neck extending into the bladder vesicle.
- Assess the trigone and bladder for any damage.

- If required, using a foreign body retrieval grasper or other applicable instrument, remove:
   - Improperly placed implants (i.e. implants placed within the bladder or exposed to stagnant urine)
   - Implants not well apposed to tissue (i.e. untensioned implants or any loose implant components/device components)

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present disclosure may be embodied as a system, method or computer program product. Accordingly, some embodiments of the present disclosure may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, some embodiments of the present disclosure may take the form of a computer program product embodied in one or more computer readable medium(s) having computer readable program code embodied thereon. Implementation of the method and/or system of some embodiments of the disclosure can involve performing and/or completing selected tasks manually, automatically, or a combination thereof. Moreover, according to actual instrumentation and equipment of some embodiments of the method and/or system of the disclosure, several selected tasks could be implemented by hardware, by software or by firmware and/or by a combination thereof, e.g., using an operating system.

For example, hardware for performing selected tasks according to some embodiments of the disclosure could be implemented as a chip or a circuit. As software, selected tasks according to some embodiments of the disclosure could be implemented as a plurality of software instructions being executed by a computer using any suitable operating system. In an exemplary embodiment of the disclosure, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the disclosure.

The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present disclosure may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present disclosure may be described below with reference to flowchart illustrations and/or block diagrams of methods, apparatus (systems) and computer program products according to embodiments of the disclosure.

It will be understood that each block of the flowchart illustrations and/or block diagrams, and combinations of blocks in the flowchart illustrations and/or block diagrams, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer and may not be feasible or practical for performing purely manually, by a human expert. A human expert who wanted to manually perform similar tasks, such as manually operate an implant device, might be expected to use completely different methods, e.g., making use of expert knowledge and/or the pattern recognition capabilities of the human brain, which would be vastly more efficient than manually going through the steps of the methods described herein.

The invention is defined by the following claims.

## Claims

1. Apparatus comprising:
a device for treatment of a urethra that is constricted due to benign prostatic hyperplasia (BPH), the device comprising:
a delivery tool (101) having a proximal portion and a distal portion, the distal portion configured to be advanced to a location in an area of the urethra that is to be treated, and the distal portion comprising:
an expandable element (400) that is configured to expand within the urethra such as to enlarge the urethra in the area to be treated, the expandable element defining an outer surface;
a tissue cutter (412) disposed outside the outer surface of the expandable element, the tissue cutter configured to form a cut in an inner surface of the urethra, subsequent to the expandable element having expanded such as to dilate the urethra; and
an implant (410) that comprises a shape-memory material, and being configured to maintain the urethra in a dilated state, by being implanted within the cut in the inner surface of the urethra, **characterised by**
the implant (410) comprising two end sections and a middle section (1202) disposed between the two end sections, and the shape memory-material being shape set such that in an unconstrained configuration of the implant, the middle section is substantially straight.

2. The apparatus according to claim 1, wherein the implant (410) is configured to be implanted within the cut in the urethra such that the middle section of the implant (410) is constrained into a curved shape.

3. The apparatus according to claim 1, wherein a length of the middle section of the implant (410) is at least 50% of a length of the implant (410).

4. The apparatus according to claim 1, wherein the implant (410) is configured to be maintained in a spiral shape, while the implant is disposed within the delivery tool (101).

5. The apparatus according to claim 1, wherein the implant (410) has a length of 40 - 80 mm,
wherein the implant (410) has a thickness of 0.01 - 1 mm, and/or
wherein the implant (410) has a width of 0.5 - 4 mm.

6. The apparatus according to claim 1, wherein the tissue cutter has a length of 3 - 10 mm.

7. The apparatus according to claim 1, wherein the tissue cutter is configured to form the cut at an angle of 90 degrees with respect to a longitudinal axis of the delivery tool.

8. The apparatus according to claim 1, wherein the tissue cutter is configured to form the cut at an angle other than 90 degrees with respect to a longitudinal axis of the delivery tool.

9. The apparatus according to claim 1, wherein the expandable element has a length of 3 - 100 mm.

10. The apparatus according to claim 1, wherein the expandable element is configured to be inflated to an internal pressure of 1- 20 atm.

11. The apparatus according to claim 1, wherein the expandable element comprises a balloon.

12. The apparatus according to any one of claims 1-11, further comprising one or more implant carrier arms (404) extending from a shaft (302) of the delivery tool (101) and being disposed outside the outer surface of the expandable element (400).

13. The apparatus according to claim 12, wherein subsequently to the tissue cutter (412) forming the cut in the inner surface of the urethra, the one or more implant carrier arms (404) are configured to release the implant (410) into the cut.

14. The apparatus according to claim 12, further comprising one or more implant holders (408) coupled to the implant carrier arms (404), and wherein:
the implant (410) is configured to be disposed between the implant carrier arm (404) and the implant holder (408) such that the implant is held in place by the implant carrier arm (404) and the implant holder (408); and
the implant holders (408) being configured to be retracted proximally with respect to the implant carrier arms (404) such that the implant (410) is released by the implant carrier arms (404) into a cut within the urethra, to thereby maintain the urethra in a dilated state.

15. The apparatus according to claim 12, wherein the delivery tool (101) further comprises an optical element (1304), and wherein the one or more implant carrier arms (404) and the expandable element (400) are configured so as to allow the optical element (1304) to visualize the urethra.

## Patentansprüche

1. Gerät, umfassend:
eine Vorrichtung zur Behandlung einer Harnröhre, die aufgrund einer gutartigen Prostatahyperplasie (BPH) verengt ist, wobei die Vorrichtung umfasst:
ein Einführinstrument (101) mit einem proximalen Abschnitt und einem distalen Abschnitt, wobei der distale Abschnitt zum Vordringen zu einer Stelle in einem zu behandelnden Bereich der Harnröhre gestaltet ist, und wobei der distale Abschnitt umfasst:
ein ausdehnbares Element (400), das zur Ausdehnung innerhalb der Harnröhre gestaltet ist, so dass die Harnröhre an dem zu behandelnden Bereich vergrößert wird, wobei das ausdehnbare Element eine Außenfläche definiert;
einen Gewebeschneider (412), der außerhalb der Außenfläche des ausdehnbaren Elements angeordnet ist, wobei der Gewebeschneider zur Bildung eines Einschnitts in einer Innenseite der Harnröhre gestaltet ist, nachdem sich das ausdehnbare Element ausgedehnt hat, so dass sich die Harnröhre ausweitet; und
ein Implantat (410), das ein Formgedächtnismaterial umfasst, und gestaltet ist, die Harnröhre in einem geweiteten Zustand zu halten, indem es in den Einschnitt in der Innenseite der Harnröhre implantiert wird, **dadurch gekennzeichnet, dass**
das Implantat (410) zwei Endabschnitte sowie einen zwischen den beiden Endabschnitten angeordneten Mittelabschnitt (1202) umfasst, und das Formgedächtnismaterial in seiner Form festgelegt ist, so dass bei einer uneingeschränkten Konfiguration des Implantats der Mittelabschnitt im Wesentlichen gerade ist.

2. Gerät nach Anspruch 1, wobei das Implantat (410) gestaltet ist, in den Einschnitt in der Harnröhre implantiert zu werden, so dass der Mittelabschnitt des Implantats (410) in eine gekrümmte Form gezwungen wird.

3. Gerät nach Anspruch 1, wobei eine Länge des Mittelabschnitts des Implantats (410) mindestens 50% einer Länge des Implantats (410) ausmacht.

4. Gerät nach Anspruch 1, wobei das Implantat (410) gestaltet ist, in Spiralform gehalten zu werden, während das Implantat in dem Einführinstrument (101) angeordnet wird.

5. Gerät nach Anspruch 1, wobei das Implantat (410) eine Länge von 40-80 mm aufweist, wobei das Implantat (410) eine Dicke von 0,01-1 mm aufweist, und/oder
wobei das Implantat (410) eine Breite von 0,5-4 mm aufweist.

6. Gerät nach Anspruch 1, wobei der Gewebeschneider eine Länge von 3-10 mm aufweist.

7. Gerät nach Anspruch 1, wobei der Gewebeschneider gestaltet ist, den Einschnitt in einem Winkel von 90 Grad in Bezug auf eine Längsachse des Einführinstruments zu bilden.

8. Gerät nach Anspruch 1, wobei der Gewebeschneider gestaltet ist, den Einschnitt in einem anderen Winkel als 90 Grad in Bezug auf eine Längsachse des Einführinstruments zu bilden.

9. Gerät nach Anspruch 1, wobei das ausdehnbare Element eine Länge von 3-100 mm aufweist.

10. Gerät nach Anspruch 1, wobei das ausdehnbare Element gestaltet ist, bis zu einem Innendruck von 1-20 atm aufgepumpt zu werden.

11. Gerät nach Anspruch 1, wobei das ausdehnbare Element einen Ballon umfasst.

12. Gerät nach einem der Ansprüche 1-11, das weiter einen oder mehrere Implantat-Trägerarme (404) umfasst, die sich von einer Welle (302) des Einführinstruments (101) erstrecken und außerhalb der Außenseite des ausdehnbaren Elements (400) angeordnet sind.

13. Gerät nach Anspruch 12, wobei im Anschluss an die Bildung des Einschnitts in der Innenseite der Harnröhre durch den Gewebeschneider (412) der eine oder mehrere Implantat-Trägerarme (404) gestaltet sind, das Implantat (410) in den Einschnitt auszuklinken.

14. Gerät nach Anspruch 12, das weiter einen oder mehrere Implantathalter (408) umfasst, die mit den Implantat-Trägerarmen (404) gekoppelt sind, und wobei:
das Implantat (410) zur Anordnung zwischen dem Implantat-Trägerarm (404) und dem Implantathalter (408) gestaltet ist, so dass das Implantat durch den Implantat-Trägerarm (404) und den Implantathalter (408) in Position gehalten wird; und
die Implantathalter (408) gestaltet sind, proximal in Bezug auf die Implantat-Trägerarme (404) zurückgezogen zu werden, so dass das Implantat (410) durch die Implantat-Trägerarme (404) in einen Einschnitt in der Harnröhre ausgeklinkt wird, um dadurch die Harnröhre in einem geweiteten Zustand zu halten.

15. Gerät nach Anspruch 12, wobei das Einführinstrument (101) weiter ein optisches Element (1304) umfasst, und wobei der eine oder mehrere Implantat-Trägerarme (404) und das ausdehnbare Element (400) derart gestaltet sind, dass sie es dem optischen Element (1304) ermöglichen, die Harnröhre zu visualisieren.

## Revendications

1. Appareil comprenant :
un dispositif pour le traitement d'un urèthre qui est rétréci en raison d'une hypertrophie bénigne de la prostate (BPH), le dispositif comprenant :
un outil de pose (101) présentant une partie proximale et une partie distale, la partie distale étant configurée pour être avancée vers un emplacement dans une zone de l'urèthre qui est à traiter, et la partie distale comprenant :
un élément expansible (400) qui est configuré pour être mis en expansion dans l'urèthre de manière à élargir l'urèthre dans la zone à traiter, l'élément expansible définissant une surface extérieure ;
un dispositif de coupe de tissu (412) disposé à l'extérieur de la surface extérieure de l'élément expansible, le dispositif de coupe de tissu étant configuré pour former une découpe dans une surface intérieure de l'urèthre, après que l'élément expansible s'est mis en expansion de manière à dilater l'urèthre ; et
un implant (410) qui comprend un matériau à mémoire de forme, et étant configuré pour maintenir l'urèthre dans un état dilaté, en étant implanté dans la découpe dans la surface intérieure de l'urèthre **caractérisé en ce que**
l'implant (410) comprend deux sections d'extrémité et une section centrale (1202) disposée entre les deux sections d'extrémité, et le matériau à mémoire de forme étant ajusté en forme de telle sorte que dans une configuration non contrainte de l'implant, la section centrale est sensiblement droite.

2. Appareil selon la revendication 1, dans lequel l'implant (410) est configuré pour être implanté dans la découpe dans l'urèthre de telle sorte que la section centrale de l'implant (410) est contrainte en une forme courbée.

3. Appareil selon la revendication 1, dans lequel une longueur de la section centrale de l'implant (410) est au moins 50 % d'une longueur de l'implant (410).

4. Appareil selon la revendication 1, dans lequel l'implant (410) est configuré pour être maintenu dans une forme en spirale, pendant que l'implant est disposé dans l'outil de pose(101).

5. Appareil selon la revendication 1, dans lequel l'implant (410) présente une longueur de 40 à 80 mm, dans lequel l'implant (410) présente une épaisseur de 0,01 à 1 mm, et/ou
dans lequel l'implant (410) présente une largeur de 0,5 à 4 mm.

6. Appareil selon la revendication 1, dans lequel le dispositif de coupe de tissu présente une longueur de 3 à 10 mm.

7. Appareil selon la revendication 1, dans lequel le dispositif de coupe de tissu est configuré pour former la découpe à un angle de 90 degrés par rapport à un axe longitudinal de l'outil de pose.

8. Appareil selon la revendication 1, dans lequel le dispositif de coupe de tissu est configuré pour former la découpe à un angle autre que 90 degrés par rapport à un axe longitudinal de l'outil de pose.

9. Appareil selon la revendication 1, dans lequel l'élément expansible présente une longueur de 3 à 100 mm.

10. Appareil selon la revendication 1, dans lequel l'élément expansible est configuré pour être gonflé jusqu'à une pression interne de 1 à 20 atm.

11. Appareil selon la revendication 1, dans lequel l'élément expansible comprend un ballonnet.

12. Appareil selon l'une quelconque des revendications 1 à 11, comprenant en outre un ou plusieurs bras porteurs d'implant (404) s'étendant à partir d'une tige (302) de l'outil de pose (101) et disposés à l'extérieur de la surface extérieure de l'élément expansible (400).

13. Appareil selon la revendication 12, dans lequel après que le dispositif de coupe de tissu (412) a formé la découpe dans la surface intérieure de l'urèthre, les un ou plusieurs bras porteurs d'implant (404) sont configurés pour libérer l'implant (410) dans la découpe.

14. Appareil selon la revendication 12, comprenant en outre un ou plusieurs supports d'implant (408) accouplés aux bras porteurs d'implant (404), et dans lequel :
l'implant (410) est configuré pour être disposé entre le bras porteur d'implant (404) et le support d'implant (408) de telle sorte que l'implant est maintenu en place par le bras porteur d'implant (404) et le support d'implant (408) ; et
les supports d'implant (408) étant configurés pour être rétractés de manière proximale par rapport aux bras porteurs d'implant (404) de telle sorte que l'implant (410) est libéré par les bras porteurs d'implant (404) dans une découpe dans l'urèthre, pour maintenir ainsi l'urèthre dans un état dilaté.

15. Appareil selon la revendication 12, dans lequel l'outil de pose (101) comprend en outre un élément optique (1304), et dans lequel les un ou plusieurs bras porteurs d'implant (404) et l'élément expansible (400) sont configurés de manière à permettre à l'élément optique (1304) de visualiser l'urèthre.
